# EUROPEAN PATENT SPECIFICATION

(11) **EP 3 253 880 B1**
(45) Date of publication and mention of the grant of the patent: **29.12.2021**
(21) Application number: 16747295.0
(22) Date of filing: 04.02.2016
(51) Int. Cl.: C12N 15/82, A01H 1/00

(54) **METHODS FOR PLASTID TRANSFORMATION**
VERFAHREN ZUR PLASTIDUMWANDLUNG
PROCÉDÉS DE TRANSFORMATION DE PLASTES

(30) Priority: 04.02.2015 US 201562111859 P
(43) Date of publication of application: 13.12.2017
(73) Proprietor: Monsanto Technology LLC, St. Louis, MO 63167 (US)
(72) Inventor: MARTINELL, Brian, J., St. Louis, MO 63167 (US); O'KEEFE, Anna, Mary, St. Louis, MO 63167 (US); SOMERS, David, Alan, St. Louis, MO 63167 (US); WILLIAMS, Edward, James, St. Louis, MO 63167 (US); YE, Xudong, St. Louis, MO 63167 (US)
(74) Representative: BIP Patents
(86) International application number: PCT/US2016/016639
(87) International publication number: WO 2016/126990

(56) References cited:
- WO-A1-2014/065857
- US-A1- 2004 133 937
- US-A1- 2004 210 955
- US-A1- 2008 280 361
- US-A1- 2008 289 063
- US-A1- 2012 001 007
- US-A1- 2014 157 453
- FRAME B R ET AL: "Production of transgenic maize from bombarded type II callus: Effect of gold particle size and callus morphology on transformation efficiency", IN VITRO CELLULAR & DEVELOPMENT BIOLOGY. PLANT, GAITHERSBURG, MD, US, vol. 36, no. 2, 1 January 2000 (2000-01-01), pages 21-29, XP002982138, ISSN: 1054-5476

### SEQUENCE LISTING

<110> Monsanto Technology LLC
<120> METHODS FOR PLASTID TRANSFORMATION
<130> MONS:371WO
<150> 62/111,859 <151> 2015-02-04
<160> 26
<170> PatentIn version 3.5
<210> 1
   <211> 221
   <212> DNA
   <213> Glycine max
<400> 1
   aacccaatgt aagtttttct atttgtatgc cgtgatcgaa taataattga gaatggataa        60
   gaggctcgtg ggattacacg aggggtgggg gggctatatt tctgggagcg aactccagtc       120
   gaatatgaag cgcctggata caagttatgc cttggaatgg aagagaattc cgaatcagct       180
   ttgtctacga acaaggaagc tataagtaat gcaactagga a                            221
<210> 2
   <211> 38
   <212> DNA
   <213> Artificial sequence
<220>
   <223> synthetic oligonucleotide
<400> 2
   tagaaataat tttgtttaac tttaagaagg agatatac **38**
<210> 3
   <211> 189
   <212> DNA
   <213> Artificial sequence
<220>
   <223> synthetic oligonucleotide
<400> 3
   gatcgaataa taattgagaa tggataagag gctcgtggga ttacacgagg ggtggggggg        60
   ctatatttct gggagcgaac tccagtcgaa tatgaagcgc ctggatacaa gttatccctt       120
   ggaatagaag acaattccgg atcctgtaga aataattttg tttaacttta agaaggagat       180
   atacccatg                                                                189
<210> 4
   <211> 119
   <212> DNA
   <213> Artificial sequence
<220>
   <223> synthetic oligonucleotide
<400> 4
   aattctgatc gaataataat tgagaatgga taagaggctc gtgggattac acgaggggtg 60
   ggggggctat atttctggga gcgaactccg tttaacttta agaaggagat atacccatg 119
<210> 5
   <211> 285
   <212> DNA
   <213> Glycine max
<400> 5
   tcgattccaa aattaaaaaa agggatagga gattaaaaat tgaaaaaaaa aaaacaaaaa 60
   ttgggttgcg ccatacatat aaaagagtat acaataatga tgtatttgac aaatcaaata 120
   ccatcgttta ataataacga accttgggga ttaattgata atattagttt attatttggg 180
   aaagattcct gtgaaatgaa agatttcatt aactcctaag tcatgtcgag tagaccttgt 240
   tgtttcgaga attcttaatt catgagttgt agggagggat ttatg 285
<210> 6
   <211> 176
   <212> DNA
   <213> Artificial sequence
<220>
   <223> synthetic oligonucleotide
<400> 6
   ggattattta gattagatat ttcgattcca aaattaaaaa aagggatagg agattaaaaa 60
   ttgaaaaaaa aaaaacaaaa attgggttgc gccatacata taaaagagta tacaataatg 120
   atgtatttga caaatcaaat accatcgttt aactttaaga aggagatata catatg 176
<210> 7
   <211> 187
   <212> DNA
   <213> Glycine max
<400> 7
   tgaaataaaa aaaagggggt gtggatgact tgtatataga tttctaaaac tcttttctct 60
   tttatccccc cgctctcttg ttctattcat acctaatttt tcttatttct tgttgtttat 120
   atagaatgat aggatgtagt tttctatagc cagaaaaaga aatgaaattt gcatcgatct 180
   tcaaaac 187
<210> 8
   <211> 219
   <212> DNA
   <213> Glycine max
<400> 8
   aattttgatc ttaaggtaga ttagtttttg aaagtaaaag gcaatatcaa cttttttgat 60
   attgcccctt tacttttatt tatttgatta gtaatctttt tatttataaa tatttataca 120
   taagtttttg atttctttcg cgattcttta gcatttttcc tatcttaaaa ggaaaaaaga 180
   atgataacga acgaaaggat agaattttat atatagatc 219
<210> 9
   <211> 62
   <212> DNA
   <213> Artificial sequence
<220>
   <223> primer
<400> 9
   cacaggaaac agctatgacc atgattacgc aagctttccc ttacgaaact ttcgttattg 60
   gg 62
<210> 10
   <211> 70
   <212> DNA
   <213> Artificial sequence
<220>
   <223> primer
<400> 10
   cagtcacgac gttgtaaaac gacggccagt gggtacccga aaattatggg aaaaattgga 60
   ttccattgtc 70
<210> 11
   <211> 78
   <212> DNA
   <213> Artificial sequence
<220>
   <223> primer
<400> 11
   gacaatggaa tccaattttt cccataattt tcgggtaccc ggagctgcag ctatccgtaa 60
   tagtgtgaaa agaaagcc 78
<210> 12
   <211> 64
   <212> DNA
   <213> Artificial sequence
<220>
   <223> primer
<400> 12
   cagtcacgac gttgtaaaac gacggccagt gatctagagg gtgtgaagca gtgtcaaact 60
   aaac 64
<210> 13
   <211> 53
   <212> DNA
   <213> Artificial sequence
<220>
   <223> primer
<400> 13
   ccaatttttc ccataatttt cgggtacccg aattcgctcc cccgccgtcg ttc 53
<210> 14
   <211> 58
   <212> DNA
   <213> Artificial sequence
<220>
   <223> primer
<400> 14
   tttcttttca cactattacg gatagctgca gcccatgaat aaatgcaaga aaataacc 58
<210> 15
   <211> 24
   <212> DNA
   <213> Artificial sequence
<220>
   <223> primer
<400> 15
   cacttgaaat tgagtatccg ttcc 24
<210> 16
   <211> 24
   <212> DNA
   <213> Artificial sequence
<220>
   <223> primer
<400> 16
   ttcctgctag gattggaaat cctg 24
<210> 17
   <211> 24
   <212> DNA
   <213> Artificial sequence
<220>
   <223> primer
<400> 17
   catatccata cgattgagtc cttg 24
<210> 18
   <211> 26
   <212> DNA
   <213> Artificial sequence
<220>
   <223> primer
<400> 18
   gctcatgagt ttagtcttac ttcacc 26
<210> 19
   <211> 24
   <212> DNA
   <213> Artificial sequence
<220>
   <223> primer
<400> 19
   agtagctcct atcaacttgt tccg 24
<210> 20
   <211> 24
   <212> DNA
   <213> Artificial sequence
<220>
   <223> primer
<400> 20
   gctctaccac tgagctaata gccc 24
<210> 21
   <211> 26
   <212> DNA
   <213> Artificial sequence
<220>
   <223> primer
<400> 21
   caacaagaat tgggacaact ccagtg 26
<210> 22
   <211> 27
   <212> DNA
   <213> Artificial sequence
<220>
   <223> primer
<400> 22
   gtgcccatta acatcaccat ctaattc 27
<210> 23
   <211> 26
   <212> DNA
   <213> Artificial sequence
<220>
   <223> primer
<400> 23
   gagcgaaatg tagtgcttac gttgtc 26
<210> 24
   <211> 26
   <212> DNA
   <213> Artificial sequence
<220>
   <223> primer
<400> 24
   tcagcccgtc atacttgaag ctagac 26
<210> 25
   <211> 21
   <212> DNA
   <213> Artificial sequence
<220>
   <223> primer
<400> 25
   ctgggattac acatggcatg g 21
<210> 26
   <211> 21
   <212> DNA
   <213> Artificial sequence
<220>
   <223> primer
<400> 26
   agcaagatag ccagatcaat g 21

## Description

### FIELD OF THE INVENTION

The present invention relates to the field of agricultural biotechnology, and more specifically to methods and compositions for genetic transformation of plastids.

### BACKGROUND OF THE INVENTION

Plastid transformation can provide significant advantages over conventional nuclear transformation methods for creating transgenic plants, including possibly more abundant and reliable transgene expression, maternal inheritance, lack of silencing mechanisms, etc. However, existing methods for plastid transformation have generally required the use of developed or callus tissues as the target for transformation and have thus been limited to certain plant species and genotypes. Furthermore, existing methods for plastid transformation have also been time-consuming and inefficient, making them impracticable for rapid, large-scale production of transplastomic plants, particularly in elite germplasms of agronomically important crops.

Improved compositions and methods are needed in the art for rapidly and efficiently producing transplastomic events, and developing or regenerating plants from those events, without requiring a callus phase, such that plastid transformation may be made feasible for large- scale commercial production of transplastomic crop plants.

### SUMMARY OF THE INVENTION

In one aspect, the present invention provides methods of transforming a plant plastid, comprising the steps of: (a) preparing an explant from a seed of a plant, the explant comprising meristematic tissue of a mature embryo of the seed; and (b) transforming at least one plastid of a cell of the explant with an exogenous DNA molecule, the exogenous DNA molecule comprising: (i) a first arm region homologous to a first plastid genome sequence; (ii) a second arm region homologous to a second plastid genome sequence; and (iii) an insertion sequence positioned between the first arm region and the second arm region of the exogenous DNA molecule, wherein the cells of the explant do not form a callus tissue prior to the transforming step (b), and wherein the insertion sequence is incorporated into a plastid genome of the plant cell between the first plastid genome sequence and the second plastid genome sequence. In certain embodiments, the seed is dry seed comprising a mature embryo. In some embodiments, the dry seed has a moisture content in a range from about 3% to about 25%. In further embodiments, the plant is a dicotyledonous plant. For example, the dicotyledonous plant may be a soybean, canola, alfalfa, sugar beet or cotton plant. In yet further embodiments, at least one plastid of the explant is not photosynthetically active.

In specific embodiments of the invention, an insertion sequence is incorporated into the plastid genome by homologous recombination. In some embodiments, the transforming step comprises introducing the exogenous DNA molecule into the explant via particle-mediated bombardment. In further embodiments, the explant prepared has a moisture content in a range from about 3% to about 20%. In yet further embodiments, the plastid transformed is a proplastid. In other embodiments, the embryo is an immature embryo. In certain embodiments, the explant remains competent for plastid transformation and does not germinate prior to the transforming step, and may be further defined as in a state of metabolic stasis. Optionally, the invention provides methods further comprising preculturing the explant prior to the transforming step. The preculturing step may comprise, for example, exposing the explant to an aqueous medium comprising at least one osmoticum. In some embodiments, the aqueous medium comprises a sugar or polyethylene glycol (PEG). In further embodiments, the invention comprises germinating the explant after the transforming step. A plastid transformed plant may also be regenerated from the germinated explant. In other embodiments, the invention provides methods further comprising obtaining a plastid transformed seed from the plastid transformed plant. In yet other embodiments, the invention provides methods further comprising germinating the explant prior to the transforming step (b).

In other embodiments of the invention, an insertion sequence comprises a DNA expression cassette comprising a transgene operably linked to a plastid promoter. The transgene may confer, for example, a trait of agronomic interest when expressed in a plant transformed with the transgene. Examples of traits of agronomic interest include modified carbon fixation, modified nitrogen fixation, herbicide tolerance, insect resistance, increased yield, fungal disease tolerance, virus tolerance, nematode tolerance, bacterial disease tolerance, modified starch production, modified oil production, modified fatty acid content, modified protein production, enhanced animal and human nutrition, environmental stress tolerance, improved processing traits, improved digestibility, modified enzyme production, and modified fiber production. The transgene may also comprise a plant selectable marker gene conferring tolerance to a selection agent and the methods may comprise selecting for development of plastid transformed cells of the explant by contacting the explant with the selection agent. In embodiments, the invention provides methods comprising developing a plastid transformed plant from the explant transformed under selection pressure by contacting the developing plant with the selectionagent.

In still other embodiments, an insertion sequence used in accordance with the invention can comprise a first DNA expression cassette and a second DNA expression cassette, the first DNA expression cassette comprising a first transgene operably linked to a first plastid promoter, and the second DNA expression cassette comprising a second transgene operably linked to a second plastid promoter, wherein the first transgene confers a trait of agronomic interest when expressed in a plant, and the second transgene is a plant selectable marker gene conferring tolerance to a selection agent. In some embodiments, a method of the invention comprises storing the explant under dry conditions for about 1 hour to about 2 years prior to the transforming step, wherein the explant remains viable and competent for transformation during storage. In further embodiments, the invention provides methods further comprising drying the explant prior to the transforming step. In yet further embodiments, the invention provides methods further comprising excising the explant from the seed of the plant.

In another aspect, the invention provides plastid transformed plants or seeds produced by the methods provided by the invention. In particular embodiments, the plant is a dicotyledonous plant. Examples of dicotyledonous plants include soybean, canola, alfalfa, sugar beet and cotton plants.

In yet another aspect, the invention provides methods of transforming a plant plastid, comprising the steps of: transforming at least one plastid of a meristematic cell of a mature embryo explant of a dry seed with an exogenous DNA molecule, the exogenous DNA molecule comprising: (i) a first arm region homologous to a first plastid genome sequence; (ii) a second arm region homologous to a second plastid genome sequence; and (iii) an insertion sequence positioned between the first arm region and the second arm region of the exogenous DNA molecule, wherein the cells of the explant do not form a callus tissue prior to the transforming step, and wherein the insertion sequence is incorporated into the plastid genome of the plant cell between the first plastid genome sequence and the second plastid genome sequence. In certain embodiments, the invention provides methods further comprising excising the explant from a seed of a plant.

### BRIEF DESCRIPTION OF THE DRAWINGS

**FIG. 1** shows soy plastid transformation vector pMON286766.
**FIG. 2** shows soy plastid transformation vector pMON285270.
**FIG. 3** shows soy plastid transformation vector pMON286706.
**FIG. 4** shows soy plastid transformation vector pMON291978.
**FIG. 5** shows an image of mature soybean embryo explants excised from dry seeds.
**FIG. 6** shows constructs used for soy plastid transformation.
**FIG. 7** shows images of soybean plant shoots from explants bombarded with the soy plastid vector pMON285270 (right column; "Event 1") versus explants blasted with a nuclear expression vector pMON96999 containing *gus* and *aadA* expression cassettes (left column; "Control Construct") under Brightfield conditions (top images), or with a "GFP2" setting on LEICA software (Excitation Filter 480/40 nm (460-500 nm); Barrier Filter (510 LP)) (middle images), or with a "GFP3" LEICA setting (Excitation Filter 470/40 nm (450-490 nm); Barrier Filter 525/50 nm (500-550 nm)) (bottom images).
**FIG. 8** shows an overview of the method for generating soy transplastomic events according to embodiments of the present invention.
**FIG. 9** shows a PCR assay for molecular analysis of putative soy plastid transformants. Dark gray fragments represent extended plastid sequences in soy chloroplast DNA after integration through homologous recombination.
**FIG. 10** shows PCR verification of plastid transformation for Event 1.

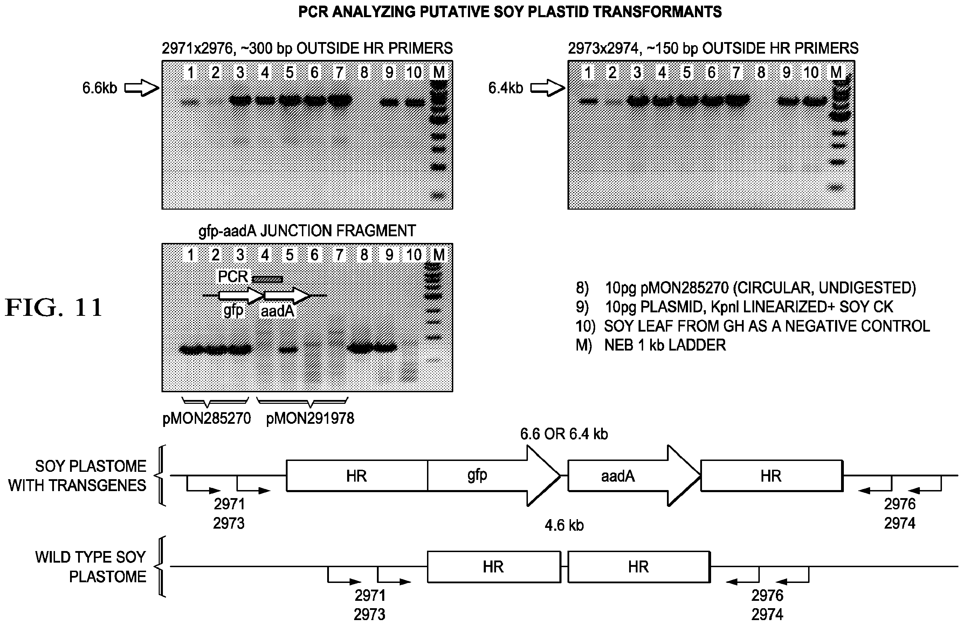

**FIG. 11** shows PCR verification of plastid transformation for Event 4, Event 5, and Event 8 (samples 1-3). Putative transformants are shown in lanes 1-7 of each image, while control lanes are shown in lanes 8, 9, and 10. Lane 8 shows 10 pg of pMON285270 (circular, undigested); lane 9 shows 10 pg of linearized plasmid DNA (digested at a *Kpn*I site adjacent to a homologue arm) mixed with non-transformed soy DNA sample (to rule out the possibility that the plastid vector comprising the transgene may anneal with native plastid genome to generate false positive PCR products even with primers outside the homology arms). No upper band product (~6.6 kb or ~6.4 kb depending on the primer pairs) was observed in control lanes. Lane 10 shows soy leaf as a negative control, and the NEB 1 kb ladder is shown in the final lane M. Samples 1, 2, and 3, which exhibited amplification products indicative of plastid transformation, were designated Event 4, Event 5, and Event 8, respectively.
**FIG. 12** shows PCR analysis of putative soy plastid transformants for the presence of a *gƒp*-HR junction fragment and an *aadA-HR* junction fragment. Samples 1, 2, and 3, designated Event 4, Event 5, and Event 8, respectively, each comprised both a *gƒp*-HR junction fragment and an *aadA-*HR junction fragment.
**FIG. 13** shows a diagram of a transformed soy plastid DNA molecule with indication of restriction sites and predicted Southern bands with an NcoI digestion of DNA from soy plastid transformants.
**FIG. 14** shows Southern blots labeled with different probes. Plastid transformants are expected to exhibit a 9,475 bp band when labeled with a PstI/XbaI probe, while wild type plants are expected to exhibit a 7,163 bp labeled band. Plastid transformants are also expected to exhibit a labeled band with an *aadA* probe.

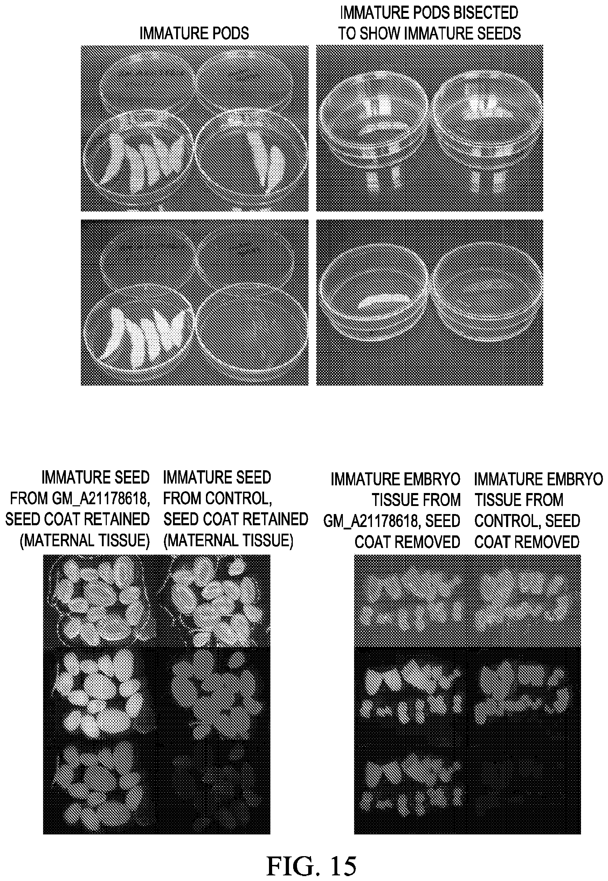

**FIG. 15** provides two sets of images showing GFP expression in transplastomic pods and seeds. The images in the top left column show GFP reporter gene expression in transplastomic immature R1 pods in comparison to control pods (see lower image; upper image is brightfield). The images in the top right column show GFP expression in transplastomic immature R1 pods and seeds (pods dissected to show seeds) in comparison to control pods (see lower image; upper image is brightfield). The sets of images in the bottom left and bottom right show GFP expression in transplastomic R1 seeds (bottom left images show immature seed with seed coat retained, whereas the bottom right images show immature seed with seed coat removed).
**FIG. 16** shows GFP quantification in transplastomic events compared with nuclear GFP event and wild type controls.
**FIG. 17** shows Droplet Digital^{™} PCR analysis of integration sites in R1 transplastomic events.
**FIGS. 18A-C** show PacBio^{®} sequencing data for R1 transplastomic events (FIGS. 18B and 18C) versus wild type controls (FIG. 18A).

### DETAILED DESCRIPTION

Plastid transformation provides a number of potential advantages over conventional nuclear transformation methods for generating transgenic plants, including generally higher levels of protein expression from transplastomic events due largely to multiple plastids being present in each cell and the presence of multiple copies of plastomic DNA molecules per plastid. Such a higher level of expression may be used to provide, for example, improved agronomic traits or increased biosynthesis of useful products. Plastids can also transcribe genes as operons, allowing for multiple transgenes or even entire pathways to be expressed together from a single expression cassette. In addition, integration of transgenes into the plastome is site-specific and generally less prone to silencing mechanisms, which may provide more consistent and reliable transgene expression levels among events for a given construct. Such consistency may thus reduce development costs in generating successful transplastomic events. Since plastids are generally maternally inherited, all R₁ seed from a plant homoplastomic for a given transgene would have the integrated transgene, unlike plants hemizygous for a nuclear transgenic event that require additional crosses to achieve stable transmission of the transgene due to chromosomal segregation. Transplastomic events may further target or sequester transgenic protein expression to plastids (or chloroplasts) which may direct or contain their function within these organelles without the need for additional target peptide sequences. As a result, plastid expression of a transgene may reduce cytotoxicity in some cases.

While attempts have been made at transforming plastids of various crop species, success has generally been limited to certain types of tissue targets, including adult leaves, protoplasts, suspension cultures, or embryogenic callus tissues. See, e.g., Bock, R., "Engineering Plastid Genomes: Methods, Tools, and Applications in Basic Research and Biotechnology," Annu. Rev. Plant Biol., 66: 3.1-3.31 (2015), However, many crop plants and cultivars are either not amenable to plastid transformation via targeting of these tissues, or unable to form callus tissue, suspension culture or protoplasts effectively. Thus, plastid transformation has generally been species and genotype dependent and often limited to more primitive cultivars of agronomically important crops, which require multiple rounds of backcrossing with more elite donor lines to achieve transgene expression in a desirable genetic background. Moreover, conventional methods of plastid transformation with plant parts, such as leaves, or callus tissues generally require extensive effort, including prolonged culturing and screening steps, to recover putative plastid transformants. As a result, these methods have been considered impracticable for large-scale commercial production of agriculturally important transplastomic crop plants. Given these limitations, existing plastid transformation methods have not provided a significant improvement over nuclear transformation despite their potential advantages and benefits, and a need therefore exists for improved methods of plastid transformation.

The present invention overcomes deficiencies in the art by providing methods for efficiently generating transplastomic events in plants by targeting cells of meristematic tissues, such as an embryonic meristem tissue, without any prior callus formation step. By avoiding the need for a callus phase prior to transformation of the targeted explant according to present methods, the genotype and species dependence experienced with existing methods may be greatly reduced or eliminated, and direct plastid transformation may thus be achieved directly into elite germplasm lines of agronomically important crop species. It has been surprisingly found that particle bombardment of meristems of plant embryo explants can be used to rapidly and efficiently produce plastid transformed Ro plants having ubiquitous transgene expression without the need for any prior callus forming step. Previously, it was believed that plastid (or proplastid) transformation could not be effectively achieved in embryonic tissues without prior callusing or tissue amplification and exposure to light because undeveloped and/or non-photosynthetic plastid targets would be too few in number and/or not amenable to transformation. In other words, plastid development and proliferation was seen as being essential for effective transformation of plastomic DNA. Surprisingly, embryo explants transformed according to methods of the present invention may be allowed to develop rather normally into adult Ro plants without a callus phase and with only minor culturing and/or regeneration steps. Transplastomic Ro plants produced by methods of the present invention are not only found to have widespread or ubiquitous transgene expression, but are further shown to be germ line transformed and able to produce transplastomic R₁ seeds and plants.

The ability to generate transplastomic Ro plants at a high frequency without extensive culturing or callusing of the explant prior to transformation allows for methods of the present invention to be carried out more rapidly and efficiently, thus enabling the potential for its implementation in large-scale, commercial production of transplastomic crop plants. Embryo explants may be taken from seeds and used almost directly as targets for transformation. According to some embodiments, embryo explants may be taken from mature dry seeds and used as targets for plastid transformation with perhaps only minimal wetting, hydration or pre- culturing steps. Accordingly, storable dry seeds or explants may be conveniently utilized as targets for plastid transformation. Alternatively, immature embryos or "wet" or "dried wet" embryo explants (including for example, primed or germinated embryo explants) may be used. Similarly, "wet excised" explants from imbibed or hydrated seeds may also be used as targets. Methods of the present invention represent a significant advance in the art because they enable rapid and efficient plastid transformation of plants, including potentially elite cultivars of agronomically important crops, without the need for additional callus formation, culturing and/or regeneration steps that would impede commercial production of transplastomic crop plants.

### I. Methods of Plastid Transformation

Embodiments of the present invention provide methods of transforming plant plastids comprising introducing an exogenous DNA molecule into at least one cell of an explant to produce a transplastomic event in at least one plastid of that cell. Methods of the present invention may be carried out by targeting the meristematic tissue or cell of an embryo explant excised from harvested seeds without extensive culturing of the explant prior to transformation. Embryo explants may include either mature or immature embryos, but may preferably include mature embryo explants excised or removed from storable, dry seeds. Methods of the present invention employ homologous recombination to achieve site-specific insertion of a transgene from an exogenous DNA molecule into the plastid genome DNA (i.e., the "plastome") of at least one cell of the explant target tissue. As described further below, the exogenous DNA molecule may generally comprise two arm regions flanking an insertion sequence with each of the two arm regions being homologous to respective plastid genome sequences to drive recombination and insertion of the transgene into the target site of the plastome. Site-directed integration of the transgene into the plastid genome of an explant cell via homologous recombination reduces event variability associated with nuclear transformation events having transgene insertions at different locations throughout the nuclear genome. As a result, transgene expression levels in plastids should generally be consistent between transplastomic events of the same quality (unlike nuclear transformation events that may exhibit variable and unpredictable levels of transgene expression depending on their insertion site). Such consistent and predictable transgene expression reduces development costs for producing transplastomic events especially if they can be produced efficiently and at a sufficient frequency.

According to some embodiments, explants excised from plant seeds may optionally be precultured in an aqueous medium for a limited time prior to transformation. Such a preculture medium may comprise various salt(s) (e.g., MS basal salts, B5 salts, etc.) and other ingredients, such as various osmoticum(s), sugar(s), antimicrobial agent(s), etc. The preculture medium may be solid or liquid and may further comprise one or more plant growth regulators or phytohormones including an auxin(s), cytokinin(s), etc. According to some embodiments, multiple explants may be precultured together in the same medium or container. For example, a range of 2-100 explants, such as about 25 or about 50 explants, may be plated on or in the same preculture medium, although a larger number of explants may be precultured together depending on the type of explant, the size of the container, dish, etc. According to some embodiments, the preculture medium may comprise an auxin, such as 2,4-D, indole acetic acid (IAA), dicamba, etc., and a cytokinin or similar growth regulator, such as thidiazuron (TDZ), 6- Benzylaminopurine (BAP), etc. Such a preculture or preculturing step may enhance the transformability and/or regenerability of the explant. Importantly, the relative amounts of auxin and cytokinin (or similar growth regulator) in the preculture medium may generally be controlled or predetermined such that callus formation from the explant is avoided (even over prolonged time periods). According to some embodiments, the preculture medium may comprise both an auxin, such as 2,4-D, and a cytokinin, such as TDZ. For example, the concentration of cytokinin in the preculture medium (if present) may be in a range from zero (0) to about 5 ppm, such as from about 0.3 to about 4 parts per million (ppm), or within any other intermediate range of concentrations. In the case of TDZ, the concentration may preferably be less than 2 ppm, or in a range from about 0.7 to about 1.3 ppm, or from about 0.5 to about 1 ppm, or about 0.5 ppm or about 1 ppm. The concentration of auxin, such as 2,4-D, may be in a range from about zero (0) to about 2 ppm, or from about 0.1 ppm to about 1 ppm, or from about 0.1 ppm to about 0.5 ppm, or within any other intermediate range of concentrations.

Depending in part on the temperature of the preculture medium and/or explant surroundings, the time period for the preculture step may vary. In general, the time period for the preculture step may also be controlled and limited to within a range from about 1 or 2 hours to about 5 days, such as from about 12 hours to about 60 hours, or from about 12 hours to about 48 hours, or any other range of time periods therein. Limiting the amount of time for the preculture step may also avoid callus formation despite the presence of plant growth regulators. During the preculture step, the explants may be kept on the same medium or transferred one or more times to a fresh medium/media. Lighting and/or temperature conditions of the optional preculture step may also be controlled. For example, the explant may be exposed to a 16/8 photoperiod exposure during the preculture step, or possibly to various other light and dark cycles or time periods. Alternatively, the preculture step may be carried out in the dark or low light conditions. Although lighted preculture conditions may promote plastid development and/or an increase the number of plastid targets in cells of the target explant tissue (conventionally thought to be necessary for plastid transformation), dark or low light preculture conditions may lead to transformation of a greater number of pro-plastids (or less developed plastids) that may proliferate to a greater extent during development of the Ro plant to provide more widespread, uniform and/or ubiquitous expression (e.g., homoplastomic expression) of the transgene in tissues of the Ro plant. The temperature of the explant preculture medium and surroundings may also vary from about 18 °C to about 35 °C, or from about 25 °C to about 30 °C, or about 28 °C, and including all intermediate ranges and values.

According to some embodiments, and regardless of whether a preculture step is performed, explant(s) for plastid transformation may optionally be exposed to a hydration or imbibition medium for a limited time prior to preculture and/or transformation. Such a hydration or hydrating step may make the explant, especially embryo explants from dry or dried seeds, more amenable to plastid transformation. Indeed, the hydration or imbibition step may be performed without a separate preculture step prior to transformation. The hydration medium may consist of only water, or may further comprise one or more known osmoticum(s), such as sugar(s) (e.g., sucrose, etc.), polyethylene glycol (PEG), etc. For example, the hydration medium may include about 10% sucrose and/or about 20% PEG. Without being bound by any theory, the osmoticum may regulate or slow the rate of hydration of the explant. Other ingredients may also be included in the hydration medium, such as various salts, etc. The time period for the hydration step may generally be short, such as from about 2 minutes to about 12 hours, or from about a 20 minutes to about 6 hours, or from about 30 minutes to about 2 hours, or for about 1 hour. The hydration or imbibition step may be short enough in time such that germination, or at least any observable germination or developmental changes, of the embryo explant does not occur. Alternatively, as discussed further below, an embryo explant may be primed for germination or even allowed to germinate prior to transformation. For example, an embryo explant may be primed for germination by wetting and then drying the explant (i.e., to produce a "dried wet" embryo explant) with arrested germination. Furthermore, a "wet excised" embryo (i.e., an embryo explant excised form a hydrated or wet seed) may also be used as a target for transformation. Before, during and/or after any of the hydration and/or preculture step(s), various rinse steps may also be performed.

According to embodiments of the present invention, the hydration and/or preculture step(s) may be included to improve transformation, especially for dry (or dried) explants, such as those taken from mature and/or dry (or dried) seeds, although either or both of these steps may be optional depending on the moisture content and/or type of explant used for plastid transformation. However, the hydration and/or preculture steps may be entirely optional and skipped (i.e., not included or performed), especially when immature embryo explants, or "wet" or "wet excised" embryo explants, are used as targets since these explants may already have a sufficient level of hydration or moisture content for effective plastid transformation. A "wet" embryo explant may be hydrated or imbibed after its excision from a seed, whereas a "wet excised" embryo explant may be excised from an already hydrated or imbibed seed.

Whether or not the hydration and/or preculture step(s) are performed, the explant is subjected to transformation with an exogenous DNA molecule to produce an explant(s) having at least one transplastomic cell. Following transformation, explant(s) may then be grown, developed, regenerated, etc., into a plant under selection pressure to select for growth and development of the transplastomic cell(s) of the explant. In general, the exogenous DNA molecule used in the plastid transformation step will contain a plastid-expressible selectable marker gene, such that survival, growth and development of transplastomic cells may be favored in the presence of a corresponding selection agent.

Various methods have been developed for transferring genes into plant tissue cells including high velocity microprojection or particle bombardment, microinjection, electroporation, PEG-mediated transformation, direct DNA uptake, and bacterially-mediated transformation. According to preferred embodiments of the present invention, an exogenous DNA molecule may preferably be introduced into at least one cell of a target explant via particle- mediated bombardment of the explant using particles carrying one or more copies of the exogenous DNA molecule. Such particle-mediated bombardment may utilize any suitable particle gun device known in the art, such as a helium particle gun, electric particle gun, etc. Prior to bombardment, particles may be loaded or coated with copies of the exogenous DNA molecule. The particles themselves may include any suitable type of particle or bead known in the art, such as gold or tungsten beads, etc. According to embodiments of the present invention, a ratio in a range of approximately 0.5 - 2.0 µg of exogenous DNA molecules per mg of beads, such as about 1.2 µ g of exogenous DNA per mg of beads, may be combined together for bead preparation and coating. Methods for coating beads with an exogenous DNA molecule are known in the art. Blasting conditions for the particle gun are also well known in the art, and various conventional screens, rupture disks, etc., may be used, such as for a helium particle gun. The electric gun may provide some advantages in reducing the amount of time required for transformation and by using fewer consumables in the process.

For particle bombardment, explants may be plated onto a target medium or substrate that is able to hold the explants in place and properly oriented for blasting. Such a target medium or substrate may contain, for example, a gelling agent, such as agar, and carboxymethylcellulose (CMC) to control the viscosity of the medium or substrate. Plating of the explants in a liquid, such as in a hydration, preculture or rinse medium, may facilitate spreading and positioning of the explants. Explants targeted for particle bombardment according to present embodiments may be positioned such that the meristematic tissue of the explant preferentially receives the particles of the blast. For example, explants may be placed on a surface with their meristems facing upward to preferentially receive the coated particles during bombardment. Each explant may also be blasted with coated particles at various pressures, forces, and/or once or multiple times.

Although particle mediated bombardment may be preferred for plastid transformation of explants according to embodiments of the present invention, other non-conventional methods are contemplated for use potentially in plastid transformation.

According to embodiments of the present invention, the targeted explant may be cultured on (or in) a post-transformation or post-culture selection medium (or a series of selection media) after transformation or bombardment that further allow at least the transgenic tissues of the explant to regenerate or develop into a plant or plant part, such as a root and/or shoot. In general, these selection media will contain a selection agent to bias or favor the survival, growth, proliferation and/or development of transplastomic cells of the explant based on the expression of a selectable marker gene from the transplastomic event (i.e., the selectable marker gene provides tolerance to the selection agent when expressed from the transplastomic event).

According to some embodiments, however, explant(s) may optionally be cultured on (or in) a first post-transformation or post-culture resting medium lacking the selection agent for a first period of time immediately following transformation or bombardment of the target explant to allow the explant to recover and/or begin to express the selectable marker gene. Such a resting step may be for a time period in a range from about one hour to about 24 hours, or form about 6 hours to about 18 hours, or from about 10 hours to about 15 hours, (e.g., about 12 hours or overnight). Although transformation frequency may be improved by having a non-selective period for recovery (e.g., culturing on a resting medium), the transformation frequency may decline if selection is initiated too late (e.g., greater than 18-24 hours after bombardment). Each of the post-culture, selection or resting media may include standard plant tissue culture media ingredients, such as salts, sugars, plant growth regulators, etc., and culturing on these media may be carried out at standard or varied temperatures (e.g., 28 °C) and lighting conditions (e.g., a 16/8 hour photoperiod). However, the first post-culture or resting step may be included or omitted prior to selection depending on the transformation and selection scheme, such as the particular selectable marker gene and selection agent used.

Following any initial recovery and culturing of the explant(s) on the first non-selective resting medium, the explant(s) may optionally undergo a plastid transformation enhancing step. According to these embodiments, the explant(s) may be exposed to, placed on (or in), etc., a second post-transformation or enhancement medium comprising an osmoticum, such as polyethylene glycol (PEG), etc., and/or a calcium-containing salt compound, such as calcium nitrate [Ca(NO₃)₂], etc. This plastid transformation enhancing medium may also lack a selection agent. For PEG-mediated plastid transformation, see, e.g., Koop, HU et al., "Integration of foreign sequences into the tobacco plastome via polyethylene glycol-mediated protoplast transformation," Planta, 199(2):193-201 (1996); Kofer W et al., In vitro Cell Dev Biol., 34(4):303-309 (1998); and Sporlein, B et al., "PEG-mediated plastid transformation: a new system for transient gene expression assays in chloroplasts," Theor Appl Genet., 82(6):717-22 (1991), For example, the concentration of calcium nitrate may be about 0.1 M, and the concentration of PEG may be about 20%, although their concentrations may vary. Exposing the bombarded explant(s) to the enhancement medium may function to further drive the coated particles and/or exogenous DNA molecule(s) into the plastids of explant cells. The explant(s) may be placed in or on the enhancement medium for only a short time period, such as in a range from about 30 minutes to about 2 hours, or for about 1 hour, which may then be followed by a rinse step(s) prior to any further culturing or selection steps. However, GFP-positive explants have been recovered by present methods without this optional plastid transformation enhancement step.

After transformation or bombardment, the explants may generally be contacted with one or more selection media containing a selection agent to bias the survival, growth, proliferation and/or development of transplastomic cells having expression of a selectable marker gene integrated into the plastome from the exogenous DNA molecule used for transformation. The selectable marker gene will generally be paired to the selection agent used for selection such that the selectable marker gene confers tolerance to selection with the selection agent. For example, the selectable marker gene may be an adenylyltransferase gene (*aadA*) conferring tolerance to spectinomycin or streptomycin as the selection agent.

To undergo the selection step(s), the explant(s) may be contacted with, or placed on (or in), one or more selection media containing a selection agent. In addition to applying the selection pressure, the selection media may simultaneously provide for the regeneration or development of shoots, roots and/or whole plants from the transformed explant(s). The selection media may contain various standard plant tissue culture ingredients, such as salts (e.g., MS or B5 salts), sugar(s), etc. The selection media may optionally include plant growth regulator(s), such as an auxin and/or a cytokinin, which may promote or assist with the development, elongation or regeneration of shoots and/or roots (and ultimately whole plants). The selection step(s) may be carried out within a range of standard or varied temperatures (e.g., 28 °C) and lighting conditions (e.g., 16/8 photoperiods). Such development of a transplastomic Ro plant on the selection media from a transformed explant may largely resemble a normal process of germination and plant development, although some reorganization of the meristem may occur in response to the selection pressure to form shoots and/or roots and other plant parts of the adult plant. Importantly, not only is a callus phase avoided before the plastid transformation step, the transformed target explant may further develop into a transgenic Ro plant without forming an embryogenic callus from the explant after transformation.

According to embodiments of the present invention, the explant(s) may be cultured in a first selection medium (or a series of selection media) until green shoots are formed, which may then be taken or cut and transferred to a new selection medium. The transferring or subculturing process may be repeated once or several times (e.g., 2, 3, 4, or 5 times) to provide multiple rounds of transfer, subculture and/or selection. It is believed that multiple rounds of transfer, subculture and/or selection of shoots from the initial explant(s) under selection pressure may expand or increase the number, proportion and/or ubiquity of transplastomic cells throughout the later developed or regenerated Ro plant.

According to some embodiments, one or more of the selection media may also function as a rooting medium to cause or allow for the formation and development of root(s) from the transferred or subcultured shoot(s). The rooting medium/media may each comprise a selection medium containing one or more plant growth regulator(s), such as an auxin and/or a cytokinin. Rooted plantlets developed or regenerated from the initially transformed explant(s) (through serial transfer or subculture under selection pressure) may eventually be transferred to PlantCon^{™} or other suitable containers and/or potted soil for the continued development of transplastomic Ro plants, and R₁ seeds may then be harvested from those Ro plants. It has been surprisingly found that only a few rounds of sequential subculturing (and eventual rooting) of green shoots derived from the initial plastid transformed explant(s) under selection pressure is sufficient to form transplastomic Ro plantlets having widespread or ubiquitous transgene expression that may be further developed into fertile plants that are able to produce transplastomic R₁ plants and seeds. The present invention represents a significant advance and improvement in the art by providing for the rapid and efficient production of transplastomic plants at a high frequency. Indeed, methods of the present invention avoid the need for a callus phase at any stage throughout the entire process of preparing the explant for transformation and then developing or regenerating a transplastomic Ro plant from the explant. In contrast to the present invention, existing methods for plastid transformation have generally been limited in their applicability to only certain crop plants and cultivars, and even when available for a particular plant species and genotype, they are generally labor-intensive and time-consuming and require extensive culturing protocols.

According to embodiments of the present invention, the selection step(s) may be performed in a single selection medium or may more preferably be carried out in a series of selection steps or media. The amount or concentration of the selection agent in a selection medium may vary depending on the particular selection agent used. For example, the amount of spectinomycin used for the selectable marker gene, *aadA,* may be in a range from about 50 ppm to about 250 ppm, or about 100 ppm or about 150 ppm. According to some embodiments, the amount or concentration of selection agent may remain constant throughout the period for selection, or the amount or concentration of selection agent may be stepped up or increased over the selection period. A stepped approach may allow more time for transplastomic explant cell(s) to recover until they can achieve a more robust expression of the selectable marker gene to withstand stronger selection pressure. However, expression of the selectable marker gene may be sufficient by the time of initial selection pressure, such that the stepped selection approach would be unnecessary. With either approach, the explant may be periodically transferred or subcultured to fresh selection media, or the selection media may be periodically replaced and refreshed with new selection media. According to some embodiments, the explant(s) may be kept in or on each of the selection media for a time period in a range from about a few days (e.g., 2 or 3 days) to several weeks (e.g., 3-4 weeks), or from about 1 week to about 3 weeks, or for about 2 weeks, before being transferred or subcultured to the next medium. According to specific embodiments involving the use of spectinomycin as the selection agent, the concentration of spectinomycin may be increased in a stepped fashion from about 50 ppm to about 500 ppm, or alternatively, the amount concentration of spectinomycin may be held relatively constant (e.g., at about 100 ppm, about 150 ppm, or about 200 ppm).

The methods of the present invention allow for more rapid regeneration and/or development of candidate transplastomic plants from one or more transformed explant(s), thus increasing the efficiency in identifying and growing plastid transformed shoots and plants, and reducing costs and labor necessary to produce transplastomic plants. For instance, after putative plastid transformants have been identified using selectable markers, plantlets may be placed in soil or on a soil substitute such as on a rooting medium, in the presence or absence of the selection agent. Shoots elongating from explants are routinely shown to be transgenic. Ro plants are further shown to give rise to transgenic R₁ plants and seeds that will likely produce subsequent progeny that are also transplastomic. The described methods thus allow for a significant decrease in the time spent under selective conditions and in usage of the selective agent, thus reducing potential costs as well. Although transplastomic Ro plants produced by methods of the present invention are shown herein to surprisingly have widespread or ubiquitous reporter transgene (e.g., GFP) expression, selection pressure with the appropriate selection agent may be optionally maintained over one or more subsequent generations from the Ro plant to produce a homoplastomic or nearly homoplastomic plant, which may be defined as being fixed or nearly fixed with respect to inheritance of the plastome-integrated transgene (i.e., without segregation of the transgene among progeny and/or with stable maintenance of homoplastomy in progeny with self-crossing). As described above, the growth, survival, development, etc., of transplastomic cells in the Ro plant may also be selectively achieved or favored by exerting a selection pressure with a selection agent during culturing, sub-culturing, shoot elongation and/or rooting step(s) of the explant to produce a homoplastomic or nearly homoplastomic Ro plant, or at least a transplastomic Ro plant having a uniform, ubiquitous or more widespread presence of transgenic plastids throughout the Ro plant, although selection pressure may alternatively be continued (e.g., periodically, etc.) during the remaining life of the Ro plant (e.g., as a topical spray, soil or seed application, etc.). Selection pressure may also be continued or maintained over subsequent generation(s) to produce a progeny plant that is homoplastomic or nearly homoplastomic, or at least has a more uniform, widespread and/or ubiquitous presence of transgenic plastids throughout the plant.

According to many embodiments, a Ro plant regenerated or developed from a plastid transformed explant according to methods of the present invention may be defined as being homoplastomic or nearly homoplastomic for a given sequence or transgene. Such a homoplastomic or nearly homoplastomic state may also be used to define any progeny plant derived from the transplastomic Ro plant produced by present methods. A perfectly homoplastomic plant or plant cell or tissue may be defined as having only transplastomic DNA in each of its plastids (i.e., 100% transplastomic). According to some embodiments, a whole plant or a plant cell or tissue may be defined as being at least 90%, at least 95%, at least 96%, at least 97%, at least 98%, at least 99%, or at least 99.5%, at least 99.9%, or 100% transplastomic. For example, a plant or plant cell or tissue that is at least 95% transplastomic is defined as a plant or plant cell or tissue with at least 95% of its plastid DNA molecules being transplastomic, as opposed to having the wild type sequence at the insertion site. Several known methods may be used to determine the percentage of plastid DNA molecules in a given plant sample that are transplastomic, such as via quantitative sequencing or PCR approaches, as well as hybridization or Southern blot analysis, or any combination thereof. See, e.g., Example 10 below. Additional statistical analysis and processing of the results may also be performed. To determine whether a whole plant is homoplastomic or nearly homoplastomic (i.e., the degree of transplastomy), one or more tissue samples may be taken from the plant, such as from one or more different locations on the plant (e.g., leaves, stem, etc.), and testing results from the individual samples may be averaged. For example, two or more tissue samples may be taken from a plant and tested to determine the percentage of transplastomic DNA for each sample individually or as an average. Thus, a plant may be defined as being at least 95% transplastomic if each of the two or more samples is at least 95% transplastomic, or if the two or more samples are at least 95% transplastomic on average.

Alternatively, one may determine a percentage of plastids in a cell or tissue having at least one transplastomic DNA molecule by detecting or observing reporter gene expression (e.g., GFP) from the transplastomic event by microscopy. To determine such a percentage, the number of transplastomic plastids or chloroplasts in a randomized set or collection, or within a field of view, could be counted and divided by the total number of plastids observed, collected, viewed, etc., within the same set, collection, or field, although this may provide a different measure than the percentage of transplastomic plastid DNA molecules in a plant cell or tissue sample.

A variety of tissue culture media are known that, when supplemented appropriately, support plant tissue growth and development, including formation of mature plants from excised plant tissue. These tissue culture media can either be purchased as a commercial preparation or custom prepared and modified by those of skill in the art. Examples of such media include, but are not limited to those described by Murashige and Skoog, (1962); Chu *et al.*, (1975); Linsmaier and Skoog, (1965); Uchimiya and Murashige, (1962); Gamborg *et al.*, (1968); Duncan *et al.*, (1985); McCown and Lloyd, (1981); Nitsch and Nitsch (1969); and Schenk and Hildebrandt, (1972), or derivations of these media supplemented accordingly. Those of skill in the art are aware that media and media supplements, such as nutrients and plant growth regulators for use in transformation and regeneration are usually optimized for the particular target crop or variety of interest. Tissue culture media may be supplemented with carbohydrates such as, but not limited to, glucose, sucrose, maltose, mannose, fructose, lactose, galactose, and/or dextrose, or ratios of carbohydrates. Reagents are commercially available and can be purchased from a number of suppliers (see, for example Sigma Chemical Co., St. Louis, MO; and PhytoTechnology Laboratories, Shawnee Mission, KS). These tissue culture media may be used as a resting media or as a selection media with the further addition of a selection agent.

A variety of assays may be performed to confirm the presence of exogenous DNA in transplastomic plants. Such assays include, for example, molecular biological assays, such as Southern and Northern blotting, sequencing, PCR, *in situ* hybridization, etc.; biochemical assays, such as detecting the presence of a protein product, e.g., by immunological means (ELISAs and Western blots) or by enzymatic function; plant part assays, such as leaf or root assays; or by analyzing the phenotype of a whole regenerated plant.

Embodiments of the present invention also provide transplastomic plants and/or plant parts produced by the plastid transformation methods of the present invention as disclosed herein. Plant parts, without limitation, include fruit, seed, endosperm, ovule, pollen, leaf, stem, and roots. In certain embodiments of the present invention, the plant or plant part is a seed.

### II. Transformable Explants

As described above, prior methods for plastid transformation generally used callus tissue, protoplasts or plant leaves (in some cases) as targets. However, plastid transformation of embryonic or shoot meristem cells to produce transplastomic plants has not been successfully reported without prior callusing or amplification of those explant tissues. Previously, it was believed that embryonic tissues were not amenable to efficient plastid transformation due to their plastids/proplastids being too few in number. Thus, amplification of explant target tissue cells by formation of a callus was believed necessary to produce a regenerated transplastomic Ro plant. In contrast, however, embryonic explants excised from plant seeds are surprisingly found to be suitable targets for plastid transformation according to methods of the present invention to produce transplastomic plants having uniform, ubiquitous or widespread transgene expression in Ro plants without undergoing any prior callus phase. In fact, transplastomic plants may be produced (i.e., developed or regenerated) from embryo explants without any callus forming step either before or after transformation. The present inventors have further shown that methods of the present invention are effective in transforming plastids of explants even prior to greening of the target explant tissue. Indeed, explants used with methods of the present invention may comprise transformable plastids or pro-plastids that may not yet be photosynthetically active. Thus, explants suitable for use in methods of the present invention may generally include plant embryo explants (i.e., explants excised from plant seeds and containing at least a portion of a plant embryo).

Methods of the present invention may further comprise step(s) for excising, or excision of, at least a portion of a plant embryo from a plant seed by any suitable manual or automated method prior to their transformation. According to embodiments of the present invention, suitable embryo explants further comprise a meristem or meristematic tissue of the embryo, or at least a portion of the meristem, or at least one meristematic cell of the embryo explant, since targeting of the meristematic cells of an explant for transformation is believed necessary for effective plastid transformation and development or regeneration of a transplastomic plant. An embryo explant may lack one or more embryonic tissues, such as cotyledon(s), hypocotyl(s) and radical, as long as it retains at least a portion of the embryo meristem. Plant embryo explants suitable for use as targets for plastid transformation methods of the present invention include mature plant embryos, or at least a portion(s) thereof, containing at least one meristematic cell or tissue. Suitable explant targets for plastid transformation may further include "wet", "dry", "wet-excised", or "dried wet" embryo explants, or a primed or germinated embryo explant, or at least a portion(s) thereof containing at least one meristematic cell or tissue. Indeed, any suitable embryonic explant may be used according to embodiments of the present invention. Use of mature embryo explants excised from dry seeds according to many embodiments of the present invention may especially require hydration and/or preculture step(s) prior to transformation.

Any suitable method for producing or excising embryo explants from plant seeds may be used in conjunction with embodiments of the present invention. These methods may be automated and/or performed manually and may involve a singulated or bulk process. According to many embodiments, the embryo explant may be a mature embryo explant (or portion thereof) taken or excised from a dry mature plant seed. For any given species of plant, a mature seed or embryo may be defined in terms of being greater than or equal to a certain number of days after pollination (DAP) to distinguish an immature seed or embryo of the same species of plant, although the transition from an immature to a mature embryo for a given plant species may be gradual and somewhat overlapping in time. In general, the transition from immature to mature embryo is accompanied by a natural process of drying or dehydration of the seed and embryo (in addition to other developmental changes) as known in the art.

Since development or maturation of a seed and embryo is accompanied by drying, a mature seed, embryo or embryo explant used in methods of the present invention may also be defined in terms of its moisture content. For example, a seed or embryo explant used according to present methods may initially have a moisture content at or within a range from about 3% to about 25%, or from about 4% to about 25%, or from about 3% or 4% to about 20%, or at or within any percentage value or range within such broader percentage ranges, depending on the particular species of plant, such as from about 5% to about 20%, about 5% to about 15%, about 8% to about 15% and about 8% to about 13%. Indeed, a plant seed may be artificially dried or dehydrated prior to excision of an embryo explant prior to use in method embodiments of the present invention as long as the seed and embryo remain viable and competent for plastid transformation. Drying of a seed may facilitate excision and/or storage of an embryo explant from the seed. Alternatively or additionally, a seed may be hydrated or imbibed prior to excision of an explant, such as to facilitate, soften, reduce damage to, and/or maintain viability of the embryo during the excision step. However, hydration of a seed or explant may reduce or eliminate the storability of the seed or explant, even if the seed or explant is subsequently dried or dehydrated.

For a further description of embryo explants and methods for excising embryo explants from dry, dried, and/or mature seeds, which may be previously hydrated, primed or germinated, see, e.g., U.S. Patent Nos. 8,466,345, 8,362,317, and 8,044,260, Regardless of the type of seeds used and the precise method for mechanically excising embryo explants from the seeds, additional steps and processes, such as sterilization, culling, etc., may also be performed to prepare and/or enrich the explants used for plastid transformation. Dry or dried embryo explants may also be hydrated, primed, and/or germinated after their excision but prior to transformation.

Embryonic explants used with the invention may have been removed from seeds less than a day prior to use in present methods, such as from about 1 to 24 hours prior to use, including about 2, 6, 12, 18 or 22 hours before use. According to other embodiments, however, seeds and/or explants may be stored for longer periods, including days, weeks, months or even years prior to their use, depending upon storage conditions used to maintain seed and/or explant viability. An advantage and benefit of using dry mature seeds as a source for producing or excising embryo explants suitable for plastid transformation, is that the dry mature seeds and/or explants may be storable (i.e., not germinating and remaining viable and competent for transformation during storage) under dry conditions. Such dry storage conditions may be defined as being stored in an environment or surroundings having a sufficiently low moisture level or humidity, such that the stored seeds and/or explants do not germinate and remain viable and competent for plastid transformation for a desired length of time prior to use in present transformation methods, such as from about 1 hour to about 2 years, or from about 24 hours to about 1 year, or for any particular period of time or range of time periods within those broader ranges of time. By using a storable seed or explant, a reliable supply of seed or explant source material may be available without the need for donor plants. The ability to store mature dry seed relates to a natural property of dry mature seeds and embryos. In other words, a dry mature seed and/or embryo explant may also be defined in terms of its quiescence, stasis or low metabolic state or activity. Thus, the dry seed or explant used according to methods of the present invention may be defined in terms of its low metabolic state and/or by its state of metabolic or developmental quiescence or stasis until later hydration and germination of the seed or embryo.

Suitable embryo explants for use in method embodiments of the present invention may further include explants comprising at least a portion of an immature embryo and/or explants excised from seeds by a "wet" process, such as from "wet" seeds. Since the present inventors have now shown that successful plastid transformation can be achieved using excised plant embryo explants as targets without any prior callus phase or greening of the explant target tissue, it is further contemplated that immature embryo explants, and even germinated embryo explants, may also be used as targets for transformation. Methods for excising embryo explants from wet, immature and/or germinated seeds are known in the art. See, e.g., U.S. Patent Nos. 8,466,345, 7,694,457, 7,658,033, and 7,402,734, In addition to meristems of embryo explants, it is further contemplated that early shoot buds or shoot apical meristems emerging or growing from a developing embryo might also be targeted for plastid transformation according to the present methods.

According to some embodiments, hydration or germination of an embryo explant or seed may be performed either before or after excision of the embryo explant from a seed. In other words, apart from any preculturing step, a seed may be imbibed or hydrated to allow the seed to begin germination and/or development prior to excision of the embryo explant, or a dry embryo explant may alternatively be excised from a seed and then imbibed or hydrated to trigger germination and/or development of the embryo explant. The primed or germinated seed may then be subjected to transformation without prior greening of the target tissue, which may be controlled by the amount of time and/or limited exposure to light prior to the plastid transformation step. However, as described above, a hydration step may instead be used only to hydrate a dry embryo explant to make the "wetted" explant more amenable to transformation without germination or further development of the embryo (e.g., the hydration or imbibition step may be limited in time such that noticeable developmental changes and/or germination of an embryo explant does not occur prior to transformation).

Explants for use with the method embodiments provided herein may include explants from a wide variety of dicotyledonous (dicot) plants including agricultural crop species, such as cotton, canola, sugar beets, alfalfa, soybean, and other Fabaceae or leguminous plants.

### III. Constructs for Plastid Transformation

### A. Transformation Vectors and Molecules

An exogenous DNA molecule is generally used for plastid transformation of a target explant tissue according to embodiments of the present invention. The exogenous DNA molecule may comprise a linear or circular DNA molecule, although circular DNA plasmids, vectors or constructs may be preferred. Vectors and molecules for plastid transformation according to methods of the present invention may comprise one or more genetic elements or transgenes to be introduced into the plant cell or tissue, which may include a selectable marker gene and/or a gene of agronomic interest. These genetic element(s) or transgene(s) may be incorporated into a recombinant, double-stranded plasmid or vector DNA molecule that may generally comprise at least the following components: (a) an insertion sequence comprising at least one transgene or expression cassette; and (b) two homology arms (derived from, and corresponding to, plastid genome sequences of the plant species to be transformed) flanking the insertion sequence. Each of the at least one transgene(s) or expression cassette(s) of the insertion DNA sequence may further comprise (i) at least one promoter or regulatory element that functions in plant cells, and more particularly in plant plastids, to cause or drive expression of a transcribable nucleic acid sequence operably linked to the promoter, and (ii) a transcribable nucleic acid sequence encoding a selectable marker or a gene product of agronomic interest (i.e., a selectable marker gene or gene of agronomic interest). The at least one transgene(s) or expression cassette(s) of the insertion sequence may further comprise a 5' and a 3' untranslated DNA sequences to provide additional elements required or beneficial for transgene expression in a plant plastid.

In general, the insertion sequence between the homology arm region(s) will at least comprise a plant selectable marker transgene since selection pressure with a corresponding selection agent is generally believed to be essential or highly desired for successful generation of plastid transformants. However, additional transgene(s) may also be present within the insertion sequence and inserted into the targeted plastid DNA molecule along with the selectable marker gene, which may include one or more transgenes of agronomic interest conferring one or more agronomically or industrially desirable traits. For example, the transgene of agronomic interest may confer one or more of the following traits: modified carbon fixation, modified nitrogen fixation, herbicide tolerance, insect resistance, improved or increased yield, fungal disease tolerance, virus tolerance, nematode tolerance, bacterial disease tolerance, modified starch production, modified oil production, modified fatty acid content, modified protein production, enhanced animal and human nutrition, environmental stress or drought tolerance, improved processing traits, improved digestibility, modified enzyme production, modified fiber production, etc. An exogenous plasmid or DNA molecule may further comprise other sequence elements required for maintenance of the exogenous DNA molecule or vector, such as a bacterial replication origin, bacterial selection marker, etc., such as in the vector backbone (e.g., outside the homology arms and insertion sequence). Means for preparing DNA plasmids, constructs or vectors containing desired genetic components and sequences are well known in the art.

### B. Homology Arms

An exogenous DNA molecule of the present invention may comprise at least two homology arms for homologous recombination at a particular site or locus within a plastid or plastomic DNA molecule of a target explant cell. The exogenous DNA molecule may comprise a first homology arm (or left homology arm) and a second homology arm (or right homology arm) flanking an insertion sequence between the left and right homology arms. Each of these homology arms may typically have a base pair (bp) length of up to about 5 kilobases (kb), such as in a range from about 0.1 kb to about 5 kb in length, or in a range from about 0.5 kb to about 2 kb in length, or in a range from about 1 kb to about 1.5 kb in length. The homology arms are positioned on either side of an insertion sequence comprising one or more transgene(s) for insertion into a plastid or plastomic DNA molecule. Each of the homology arms may generally be highly homologous, nearly identical or identical to a corresponding target plastid DNA sequence present in an explant cell, which may be known or determined empirically. For example, each homology arm may be at least 80% identical to the corresponding target plastid DNA sequence, or at least 90% identical, or at least 95% identical, etc., although lower percentages of identity are also possible. However, except in cases where a targeted mutation or editing of the plastid genome sequence is desired, the homology arms will generally be perfectly or 100% identical to the corresponding target plastid DNA sequences to improve plastid transformation efficiency and avoid introduction of additional mutations.

In addition to the homology arms being identical or highly homologous to corresponding target plastid DNA sequences, the corresponding target plastid DNA sequences will also generally be perfectly or almost perfectly continuous with each other prior to the transformation and insertion event (i.e., prior to the insertion sequence of the exogenous DNA molecule becoming inserted into the plastid genome) to avoid making any additional changes or mutations to the plastid DNA sequence as a result of the plastid transformation event. The junction of the corresponding target plastid DNA sequences will also generally or preferably be within an intergenic region or sequence of the plastid DNA to avoid insertion of a transgene into a plastid gene or coding sequence. However, each of the homology arms may comprise or encompass one or more plastid genes, or a portion(s) thereof, within their sequence. According to alternative embodiments, it is further contemplated that the target plastid DNA sequences corresponding to the homology arms may not be continuous with each other (prior to the transformation event), such that the intervening sequence will be deleted by the transformation event and replaced with the exogenous insertion sequence. This approach could thus be used to delete a portion(s) of the plastid genome and/or knockout gene(s) by the transformation event in addition to inserting the exogenous insertion sequence.

Although generally less preferred, it is conceivable that an exogenous DNA molecule used for plastid transformation according to present method embodiments may comprise only one homology arm immediately adjacent or next to an insertion sequence comprising one or more transgene(s), such as a plant selectable marker gene and/or a transgene of agronomic interest. However, having only one homology arm is generally much less preferred since it may lead to further integration of the vector backbone and/or variable event quality. Even if a linear exogenous DNA molecule is used that lacks additional unwanted vector sequences, such as a bacterial replication origin, selectable marker, etc., such an exogenous DNA molecule may have a much lower transformation frequency and variable event quality. Accordingly, two homology arms flanking the insertion sequence comprising one or more transgene(s) will generally be preferred to provide a higher transformation efficiency and greater fidelity among transformation events from an exogenous DNA molecule or construct.

According to some embodiments of the present invention, constructs and methods may be further used to engineer, create or introduce one or more mutations (e.g., point mutations or SNPs, deletions, additions, etc.) in the targeted plastid DNA molecule (with or without the additional insertion a gene of agronomic interest). In such a case, the one or more desired mutations relative to the target plastid DNA sequence may be incorporated into one or both of the homology arm(s) of the exogenous DNA molecule such that those mutation(s) may become introduced into the plastid DNA molecule via the homologous recombination event. Despite the possible absence of a gene of agronomic interest within exogenous DNA molecules used for sequence editing or creation of targeted mutations, a plant selectable marker gene may still be present between the two homology arms of the exogenous DNA sequence to allow for selection of transformed cells, tissues and plants with a selection agent. According to other embodiments, a targeted deletion or knockout of an endogenous plastid genome sequence, which may include one or more plastid gene(s), or one or more portion(s) thereof, may also be carried out by the two homologous arms having corresponding plastid target DNA sequences that are not continuous and are separated from each other in the non-transformed plastid genome.

### C. Transgene Expression Cassettes

According to embodiments of the present invention, an exogenous DNA molecule for plastid transformation may generally comprise an insertion sequence comprising one or more transgene(s), transcribable nucleic acid sequence(s), and/or expression cassette(s) that is/are introduced into a plastid DNA molecule (i.e., a plastid genome or plastome) of a plant or plant cell. Each transgene, expression cassette, etc., will generally comprise a sequence encoding a gene product of agronomic interest and/or a plant selectable marker gene, which may each be operably linked to one or more regulatory element(s), such as promoters, enhancers, leaders, introns, linkers, untranslated regions, termination regions, etc., that are suitable for regulating plastid expression of the transgene or expression cassette. In addition to the regulatory elements and promoters described herein, such as the P-rrn and rbcL promoters, other known examples of plastid regulatory elements may be operably linked to a transgene or plant selectable marker gene, and that are suitable for expression in plant plastids. See, e.g., Kung, SD et al., "Chloroplast promoters from higher plants", Nucleic Acids Res., 13(21): 7543-9 (1985); and Liere, K et al., "The transcription machineries of plant mitochondria and chloroplasts: Composition, function, and regulation", Journal ofPlant Physiology, 168: 1345-1360 (201). Plastid regulatory elements or promoters may include those naturally occurring in plastids of the plant species to be transformed, or possibly DNA sequences homologous to those plastid regulatory elements or promoters, or possibly even heterologous plastid regulatory elements or promoters from other closely, or even distantly, related species. Plastid regulatory elements and promoters may further include synthetic or engineered promoters, as well as promoters altered or derived from other regulatory element or promoter sequences.

For purposes of the present invention, the term "heterologous" means that the plastid promoter, regulatory element, transgene, selectable marker gene, etc., is from a different species than the plant species to be transformed. Thus, a plastid promoter or regulatory element in exogenous DNA molecules of the present invention may include a homologous, heterologous, or even disparate or divergent plastid or regulatory element sequence(s), in addition to nucleotide sequence(s) identical to a plastid promoter or regulatory element sequence(s) from the plant species to be transformed. A plastid regulatory element or promoter may functionally include any nucleotide sequence element that drives, or at least affects, expression of a transgene operably linked to the regulatory element or promoter (at least transiently) when the plastid regulatory element or promoter and transgene are inserted or integrated into the plastid genome of the plant species to be transformed. Even if a plasmid promoter or regulatory element from the plant species to be transformed is used, the plasmid promoter or regulatory element may be operably linked to a transgene, transcribable nucleotide sequence, selectable marker gene, etc., in a manner, form or combination that (in terms of its exact nucleotide sequence) does not naturally exist in nature, or at least does not naturally exist in the plant species to be transformed.

### D. Transcribable Nucleic Acid Sequences

The transcribable nucleic acid sequence of a transgene or expression cassette within the insertion sequence of an exogenous DNA molecule to be inserted into the plastid genome or plastomic DNA of target explant cells may include a gene of agronomic interest to be expressed in a transplastomic cell or plant. As used herein, the term "gene of agronomic interest" refers to any transgene or expression cassette comprising a transcribable nucleic acid or DNA sequence operably to one or more plastid regulatory element(s) that, when expressed in a plastid of a transgenic plant tissue or cell, provides or confers an agronomically beneficial trait or phenotype, such as a desirable product or characteristic associated with plant morphology, physiology, growth, development, yield, nutritional profile, disease or pest resistance, and/or environmental or chemical tolerance. In some embodiments, a trait of agronomic interest may be modified carbon fixation, modified nitrogen fixation, herbicide tolerance, insect resistance or control, modified or increased yield, fungal disease tolerance or resistance, virus tolerance or resistance, nematode tolerance or resistance, bacterial disease tolerance or resistance, modified starch production, modified oil production, modified fatty acid content, modified protein production, enhanced animal and human nutrition, environmental stress tolerance, improved processing traits or fruit ripening, improved digestibility, improved taste and flavor characteristics, modified enzyme production, modified fiber production, synthesis of other biopolymers, peptides or proteins, biofuel production, etc.

A gene or transgene of agronomic interest may further include a gene or transcribable DNA sequence of interest that may have unknown characteristics but may be in testing or proposed or theorized for providing a desirable trait of agronomic interest to a plant. Indeed, a transgene of agronomic interest may include any known gene (or any putative or annotated gene sequence) believed, or tested or screened for its ability, to cause, confer, or create a trait or phenotype of agronomic or industrial interest in the transplastomic plant. A transgene of agronomic interest may further include any transcribable DNA sequence that produces a desirable effect in a plant, such as RNA molecule(s) used to confer insect resistance, etc.

Examples of genes of agronomic interest known in the art may include any known or later discovered genes, coding regions or transcribable DNA sequences providing herbicide resistance or tolerance, increased yield, insect resistance or control, fungal disease resistance, virus resistance, nematode resistance, bacterial disease resistance, plant growth and development, starch production, modified oils production, high oil production, modified fatty acid content, high protein production, fruit ripening, enhanced animal or human nutrition, biopolymers, environmental stress resistance, pharmaceutical peptides and secretable peptides, improved processing traits, improved digestibility, low raffinose, industrial enzyme production, improved flavor, nitrogen fixation, hybrid seed production, fiber production, biofuel production, etc.

Plastids can be transformed with polycistronic operons, and can effectively integrate and express large transgenic inserts, thereby enabling stacking of genes and/or simultaneous expression of genes from the same insertion sequence inserted into the plastid DNA by methods of the present invention. As mentioned above, transgenes integrated in plastomes are also generally not susceptiable to gene silencing, which often occurs with multi-copy nuclear events. Thus, plastid transformation according to methods of the present invention may be particularly useful in cases in which high levels of transgene expression is desirable and/or where multiple genes or possibly even entire pathways (or portions of a biochemical pathway) need to be expressed. Accordingly, the insertion sequence of an exogenous DNA molecule may comprise (i) multiple transgenes or cassettes under the control of separate regulatory element(s), and/or (ii) a single transgene or cassette that simultaneously encodes a polycistronic RNA molecule under the control of a common set of regulatory element(s). Such insertion sequences may thus be used to produce multiple gene products from a single plastid DNA insertion event.

### E. Selectable markers

According to embodiments of the present invention, the insertion sequence of an exogenous DNA molecule for plastid transformation will generally comprise at least a plant selectable marker gene to allow for successful selection for, and production of, transplastomic Ro plants. A plant selectable marker gene or transgene may include any gene conferring tolerance to a corresponding selection agent, such that plant cells transformed with the plant selectable marker transgene may tolerate and withstand the selection pressure imposed by the selection agent. As a result, transplastomic cells of an explant are favored to grow, proliferate, develop, etc., under selection. Although a plant selectable marker gene is generally used to confer tolerance to a selection agent, additional screenable marker gene(s) may also be used in addition to the selectable marker, perhaps also along with a gene of agronomic interest. Such screenable marker genes may include, for example, β-glucuronidase (GUS; e.g., as described in U.S. Pat. No. 5,599,670,) or green fluorescent protein and variants thereof (GFP described in U.S. Pat. Nos. 5,491,084 and 6,146,826,). Additional examples of screenable markers may include secretable markers whose expression causes secretion of a molecule(s) that can be detected as a means for identifying transformed cells.

A plant selectable marker gene may comprise a gene encoding a protein that provides or confers tolerance or resistance to an herbicide, such as glyphosate and glufosinate. Useful plant selectable marker genes known in the art may include those encoding proteins that confer resistance or tolerance to streptomycin or spectinomycin (e.g., *aadA*, *spec*/*strep*), kanamycin (e.g., *nptII*), hygromycin B (e.g., *aph IV*), gentamycin (e.g., *aac3* and *aacC4*), and chloramphenicol (e.g., *CAT*). Additional examples of known plant selectable marker genes encoding proteins that confer herbicide resistance or tolerance include, for example, a transcribable DNA molecule encoding 5-enolpyruvylshikimate-3-phosphate synthase (EPSPS for glyphosate tolerance; e.g., as described in U.S. Pat. Nos. 5,627,061; 5,633,435; 6,040,497; and 5,094,945,); a transcribable DNA molecule encoding a glyphosate oxidoreductase and a glyphosate-N-acetyl transferase (GOX; e.g., as described in U.S. Pat. No. 5,463,175; GAT described in U.S. Patent publication No. 20030083480; a transcribable DNA molecule encoding phytoene desaturase (*crtI* ; e.g., as described in Misawa, et al., Plant Journal, 4:833-840 (1993) and Misawa, et al., Plant Journal, 6:481-489 (1994) for norflurazon tolerance,); and the *bar* gene (e.g., as described in DeBlock, et al., EMBO Journal, 6:2513-2519 (1987) for glufosinate and bialaphos tolerance,). See also, e.g., Bock, R., "Engineering Plastid Genomes: Methods, Tools, and Applications in Basic Research and Biotechnology," Annu. Rev. Plant Biol., 66: 3.1-3.31 (2015),

The insertion sequence of an exogenous DNA molecule may further comprise sequences for removal of one or more transgene(s) or expression cassette(s), such as a plant selectable marker transgene, or any portion or sequence thereof, after successful production and/or confirmation of a transplastomic plant(s), especially after the transgene or expression cassette is no longer needed. In some embodiments, this may be accomplished by flanking the transgene sequence to be removed, with known of later developed recombination sites (e.g., LoxP sites, FRT sites, etc.) that can be recognized and removed by an endogenous or exogenously provided recombinase enzyme (e.g., Cre, Flp, etc.). The recombinase enzyme may be introduced and expressed in trans, such as by crossing the transplastomic plant to another plant having the recombinase transgene, to accomplish excision of the transgene. Accordingly, the unwanted sequence element or transgene can be removed once its use or purpose has expired, thus preventing its further expression or transmission in the germ line.

### IV. Definitions

The following definitions are provided to define and clarify the meaning of these terms in reference to the relevant embodiments of the present invention as used herein and to guide those of ordinary skill in the art in understanding the present invention. Unless otherwise noted, terms are to be understood according to their conventional meaning and usage in the relevant art, particularly in the field of molecular biology and plant transformation.

The term "callus" refers to a dedifferentiated proliferating mass of cells or tissue.

An "embryo" is a part of a plant seed, consisting of precursor tissues (e.g., meristematic tissue) that can develop into all or part of an adult plant. An "embryo" may further include a portion of a plant embryo.

A "meristem" or "meristematic tissue" comprises undifferentiated cells or meristematic cells, which are able to differentiate to produce multiple types of plant parts, tissues or structures, such as a shoot, stem, root, leaf, seed, etc.

A "plastid" refers to a class of organelles in the cytoplasm of a plant cell which contain one or more small circular double-stranded DNA molecules (i.e., the plastome, plastomic DNA, or plastid DNA). Examples of plastids include, but are not limited to, proplastids, chloroplasts, chromoplasts, gerontoplasts, leucoplasts, elaioplasts, proteinoplasts, and tannosomes.

The term "regeneration" refers to the process of growing a plant from a plant cell.

In some embodiments, the terms "a" and "an" and "the" and similar references used in the context of describing a particular embodiment (especially in the context of certain of the following claims) can be construed to cover both the singular and the plural, unless specifically noted otherwise. In some embodiments, the term "or" is used herein to mean "and/or" unless explicitly indicated to refer to alternatives only or the alternatives are mutually exclusive.

The terms "comprise", "have" and "include" are open-ended linking verbs. Any forms or tenses of one or more of these verbs, such as "comprises", "comprising", "has", "having", "includes" and "including" are also open-ended. For example, any method that "comprises", "has" or "includes" one or more steps is not limited to possessing only those one or more steps and can also cover other unlisted steps. Similarly, any composition or device that "comprises", "has" or "includes" one or more features is not limited to possessing only those one or more features and can cover other unlisted features.

All methods described herein can be performed in any suitable order unless otherwise indicated herein or otherwise clearly contradicted by context. The use of any and all examples, or exemplary language (e.g., "such as") provided with respect to certain embodiments herein is intended merely to illuminate the present disclosure and does not pose a limitation on the scope of the present disclosure otherwise claimed.

Having described the present disclosure in detail, it will be apparent that modifications, variations, and equivalent embodiments are possible without departing from the scope of the present disclosure as further defined in the appended claims. Furthermore, it should be appreciated that all examples in the present disclosure including the following are provided as nonlimiting examples.

### EXAMPLES

### Example 1: Vector Construction for Soy Plastid Transformation

### Chimeric Soybean 16S rDNA Promoter Construction:

A soybean 16S rDNA 5' UTR sequence (SEQ ID NO: 1) comprising a -35 motif (ATTACA) and a -10 motif (GGCTATATT) according to BPROM analysis (SoftBerry, Inc.), which has been shown to drive constitutive expression in chloroplasts, was fused to a G10 leader sequence (SEQ ID NO: 2), to construct a chimeric promoter for use in soybean plastid transformation. A P-Gm.rrn/G10L promoter sequence (SEQ ID NO: 3) from pMON45263 vector containing the 16S rDNA 5' UTR sequence fused to the G10 leader sequence was found to contain two potential promoter elements, which may result in expression of two transcripts in chloroplasts. To avoid this potential issue, the second set of -35 and -10 motifs were deleted from the pMON45263 sequence to form a modified pMON45263 construct with a P-Gm.16S rrn promoter sequence (SEQ ID NO: 4). This P-Gm. 16S rrn promoter sequence was confirmed to have a ribosomal binding site (RBS) (AGGAG) and was used in transformation constructs for soybean plastid transformation of meristem-containing explants to drive transgene expression in transplastomic cells.

### P-Gm.rbcLG10 Construction:

A soybean rbcL 5' UTR sequence (SEQ ID NO: 5) was modified to include a ribosomal binding site from a G10 leader sequence to construct a chimeric P-Gm.rbcL/G10 promoter sequence (SEQ ID NO: 6) for use in soybean plastid transformation. This promoter sequence was found to have a -35 motif (TTGCGC) and a -10 motif (GTATACAAT) (based in part on a petunia rbcL X04976 and alfalfa rbcL and BPROM prediction analysis of bacterial promoters). The P-Gm.rbcL/G10 promoter sequence was further found to have a putative RBS sequence (AGGAG) before the transcription start site.

### T-Gn.rps16 and T-Gm.psbA:

T-Gm.rps16 (SEQ ID NO: 7) and T-Gm.psbA (SEQ ID NO: 8) were amplified from soybean A3555 genomic DNA.

### Construction of pMON286766 Base Vector with Chloroplast Homology Arms:

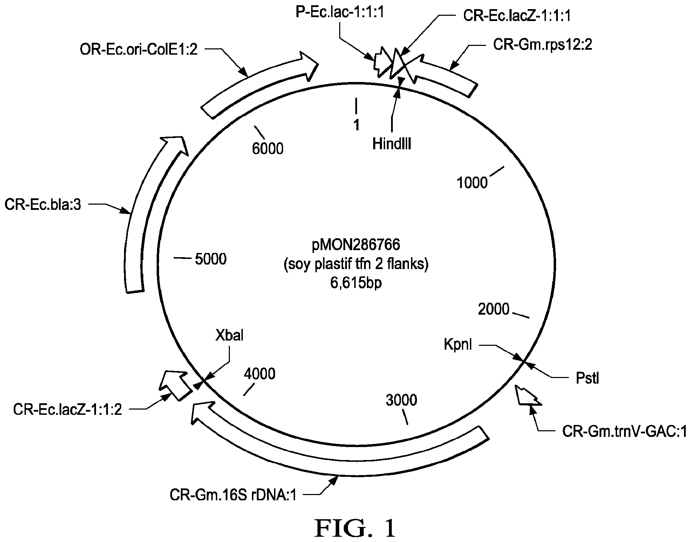

A 2 kb soybean rps12 homologue fragment was amplified from soybean genomic DNA with primers Xd2574 (SEQ ID NO: 9) and Xd2665 (SEQ ID NO: 10), and inserted into a *Hind*III/*Eco*RI site into a pUC18 plasmid. The resulting intermediate vector was opened with *Kpn*I, and a 2 kb 16S rDNA homologue fragment amplified from soybean A3555 genomic DNA using primers Xd2676 (SEQ ID NO: 11) and Xd2585 (SEQ ID NO: 12) was ligated into the open vector to produce pMON286766 (FIG. 1).

### Construction of pMON285270:

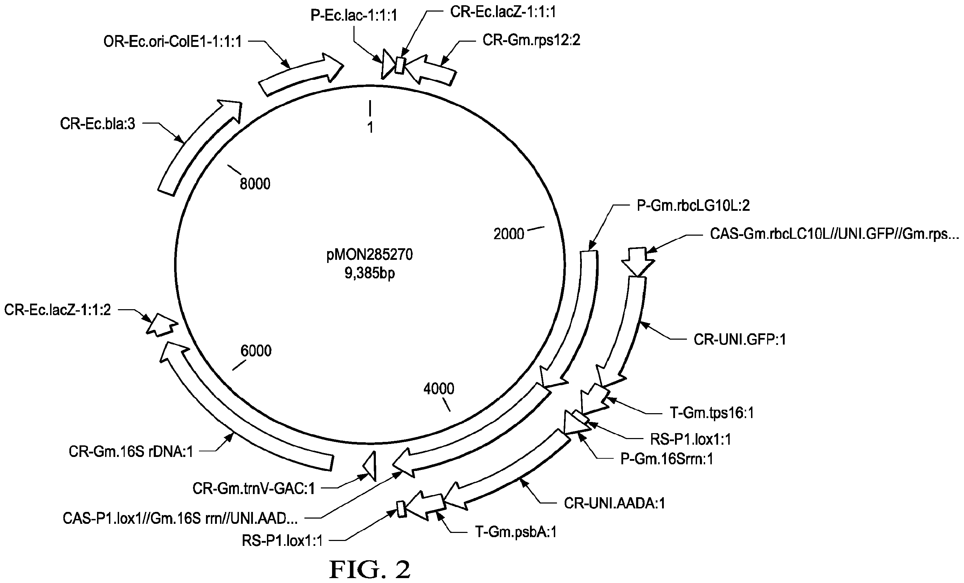

Soybean chloroplast-derived promoter sequences were synthesized by oligonucleotides, and soybean terminator sequences were amplified by PCR from soybean genomic DNA. These sequences were stitched to an *aadA* and *gfp* coding sequence fragment by PCR and inserted into a *Kpn*I/*Pst*I site in pMON286766. to produce pMON285270 (FIG. 2) as verified by sequencing.

### Construction of pMON286706:

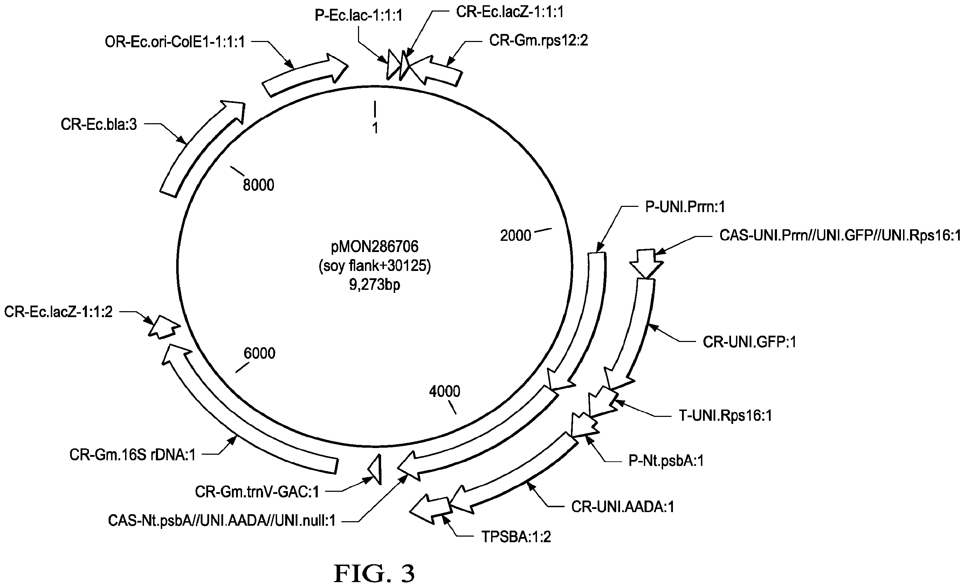

A soy plastid transformation vector, pMON286706 (FIG. 3), was constructed by moving *aadA* and *gfp* cassettes from pMON30125 (a tobacco plastid genome-derived vector; see, e.g., Sidorov, VA et al., "Technical Advance: Stable chloroplast transformation in potato: use of green fluorescent protein as a plastid marker," Plant J, 19(2):209-216 (1999),) into a *Kpn*I*lPst*I site in pMON286766. The cassettes comprising *aadA* and *gfp* sequences were amplified by PCR using primers Xd2673 (SEQ ID NO: 13) and Xd2691 (SEQ ID NO: 14).

### Construction of pMON291978:

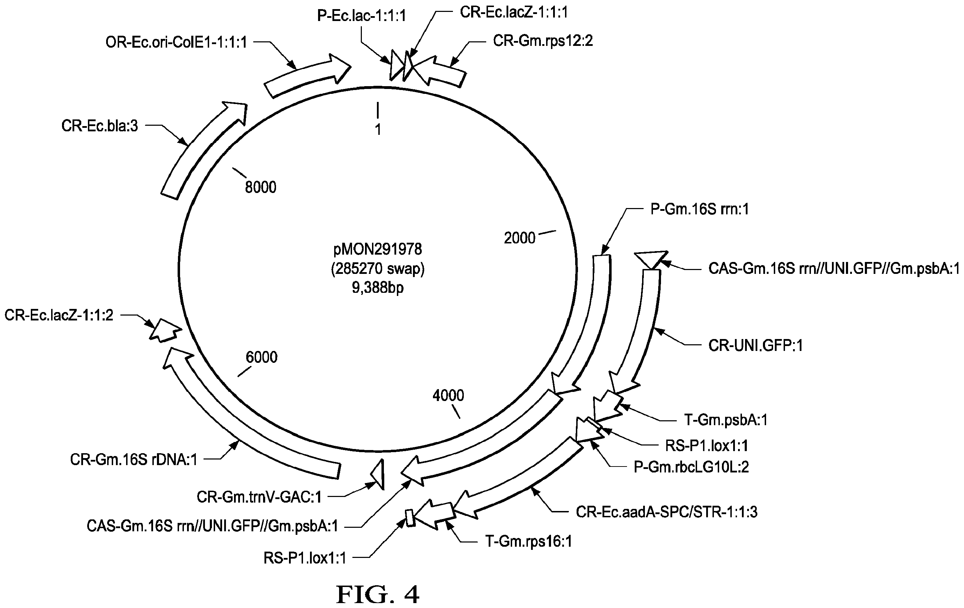

Strong GFP expression was observed in liquid and pellet of *E. coli* transformed with pMON285270, indicating strong promoter activity driving expression *of gfp* with this vector. The promoter sequences driving expression *of gfp* and *aadA* in pMON285270 were swapped to create pMON291978 (FIG. 4).

### Example 2: Preparation of Beads and

### Carrier Sheets for Bombardment of Soy Explants

Beads and carrier sheets for bombardment of dry excised embryo explants from soybean seeds using PDS1000 helium particle guns or ACCELL electric particle guns were prepared according to the following protocol.
1. 50 mg of 0.6 µm gold particles was weighed into a clean DNase, RNase free tube. Gold was washed by sonication with 1ml of 100% ethanol.
2. The gold particles were pelleted by brief centrifugation, and ethanol was completely removed.
3. The gold particles were resuspended in 1 ml of 100% ethanol, and stored at -20 °C until use. The particles were completely resuspended prior to use by sonication.
4. 42 µl of the gold particles were transferred to a new tube, pelleted by centrifugation, and the ethanol was removed.
5. 500 µl of sterilized water was added, and the gold particles were resuspended by sonication. The gold was pelleted by centrifugation, and water was removed completely.
6. 25 µl of water was added, and the gold was washed with a pipette tip before being completely resuspended by sonication.
7. DNA was added to the tube (for example, 2.6 µg DNA).
8. Ice-cold sterilized water was added immediately after adding the DNA to bring the final volume of DNA:gold particles mix to 245 µl.
9. 250 µl of ice-cold 2.5M CaCl₂ solution was added immediately.
10. 50 µl of sterilized 0.1M Spermidine was added immediately.
11. The solution was completely mixed with low speed vortexing. The tube was incubated on ice for at least 45 minutes to achieve coating of the particles. The solution may be completely mixed every 5-10 minutes for better results in some experiments.
12. The gold/DNA was pelleted by low speed centrifugation, for example by using an Eppendorf 5815 microcentrifuge at 800-1000 rpm for 2 minutes.
13. The pellet was washed with 1 ml of ethanol, and the gold particles were washed with a pipette tip and pelleted by centrifugation.
14. Ethanol was completely removed, and 36 µl of 100% ethanol was added to completely resuspend gold with low speed vortexing.
15. 5 µl of prep was used for each bombardment.
Notes:
- Sonication steps were performed at 45-55 kHz for 1 min.
- Centrifugation steps prior to step (12) above are gold washing steps - using 5000 rpm (2300g) on IEC microfuge for 10 seconds.
- Centrifugation steps (12) and (13) are post DNA coating of beads - using 1000 rpm (100 g) on IEC microfuge for 2 minutes.
- For modified electric gun prep (Accell), combine 10 of these 36 µl preps and place in scintillation vial, add 100% EtOH to 20 ml final volume.

### Example 3: Preculturing Soy Explants for Particle Bombardment

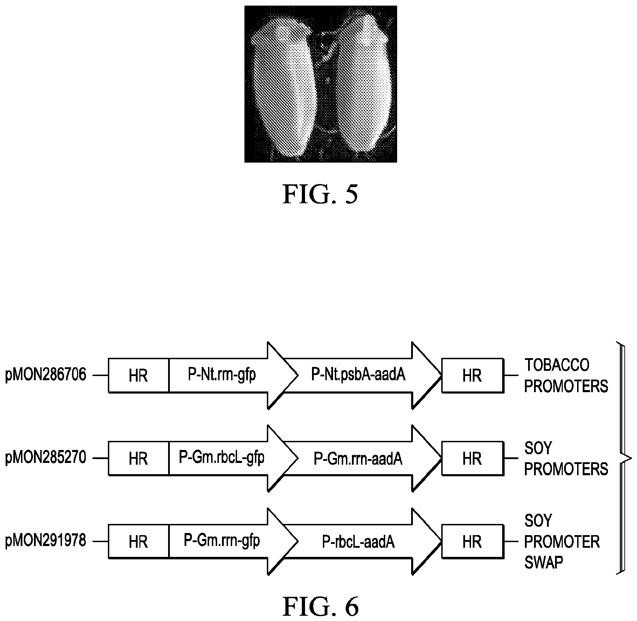

Dry excised soybean embryo explants were precultured prior to particle bombardment according to the following protocol. The mature embryo explants were excised dry soybean seeds as generally described in U.S. Patent No. 8,362,317. An example of dry excised soybean embryo explants are shown in FIG. 5 (See also, e.g., FIG. 1 of U.S. Patent No. 8,362,317).
1. The explants were weighed for blasting, rehydrated for 1 hr in either a 20% PEG4000 (Lynx 3017; Table 1) or 10% sucrose medium, and rinsed well. Lynx 1595 (Table 2) medium (or Lynx 1595 with 30 ppm Cleary's) can also be used for this step.
2. Approximately 50 explants per plate were precultured on EJW 1 media (Table 3) or EJW 2 media (Table 4). TDZ levels in the range of approximately 0.5 ppm to 2 ppm were used.
3. Explants were precultured for 1-2 days at 28 °C, either using a 16/8 photoperiod or in the dark. Preculturing the explants for 3 days was also effective.

**Table 1. Components of Lynx 3017 medium.**

| **Order of Addition** | **Ingredient** | **Amount per Liter** |
|---|---|---|
| | Measure specified amounts of ingredients below: | |
| 1 | Polyethylene glycol - PEG, MW 4000 (Sigma 81240) | 200 g |
| 2 | Add ingredient slowly, while stirring | |
| 3 | TC Water | 800 ml |
| 4 | Stir until completely dissolved | |
| 5 | Bring to volume with TC Water | |
| 6 | Autoclave at 250 °F for 25 min | |
| 7 | Antilife fungicide (1598) | 2 ml |

**Table 2. Components of Lynx 1595 medium.**

| **Order of Addition** | **Ingredient** | **Amount per Liter** |
|---|---|---|
| 1 | TC Water | 750 ml |
| 2 | B5 Stock 1 (1550) | 1 ml |
| 3 | B5 Stock 2 (1546) | 1 ml |
| 4 | B5 Stock 3 (1547) | 1 ml |
| 6 | B5 Stock 5 (1574) | 1 ml |
| 7 | Potassium Nitrate (Sigma P-8921) | 1 g |
| 8 | Glucose (Phytotech G386) | 30 g |
| 9 | MES (Sigma M-8250) | 3.9 g |
| 10 | Bring to volume with TC Water | |
| 11 | pH with KOH to | 5.4 |
| 12 | Autoclave at 250 °F for 25 min | |
| 13 | B5 Stock 4 (1551) | 1 ml |

| | | |
|---|---|---|
| B5 Stock 1: Mix 750 ml TC water, 100 g magnesium sulfate, 53.6 g ammonium sulfate, and 60 g sodium phosphate (monobasic); stir until completely dissolved and solution is clear; and bring to 1 L with TC water. B5 Stock 2: Mix 750 ml TC water and 60 g calcium chloride dihydrate; stir until completely dissolved and solution is clear; and bring to 1 L with TC water. B5 Stock 3: Mix 750 ml TC water, 0.3 g boric acid, 1 g manganese sulfate, 0.2 g zinc sulfate heptahydrate, 0.075 g potassium iodide, 0.025 g sodium molybdate dihydrate, 2.5 ml of 1 mg/ml cupric sulfate, and 2.5 ml of 1 mg/ml cobalt chloride; stir until completely dissolved and solution is clear; and bring to 1 L with TC water. B5 Stock 4: Mix 750 ml TC water, 10 g myo-inositol, 0.1 g nicotinic acid, 0.1 g pyridoxine hydrochloride, and 1 g thiamine hydrochloride; stir until completely dissolved and solution is clear; bring to 1 L with TC water; and filter sterilize with 0.22 micron unit. B5 Stock 5: Mix 750 ml TC water and 2.8 g sequestrene; stir until completely dissolved and solution is clear; and bring to 1 L with TC water. | | |

**Table 3. Components of EJW 1 media.**

| **Order of Addition** | **Ingredient** | **Amount per Liter** |
|---|---|---|
| 1 | MS basal salts (Phytotech M524) | 4.33 g |
| 2 | Sucrose (Phytotech S391) | 30 g |
| 3 | 2,4 D (1mg/ml) | 0.2 ml |
| 4 | MES | 2.0 g |
| 6 | Cleary's 3336 WP (dimethyl 4,4'-o-phenylenebis[3-thioallophantane]) | 0.03 g |
| 7 | Stir until completely dissolved and solution is clear | |
| 8 | Add TC water to bring to this volume | 1000 ml |
| 9 | pH w/ KOH to | 5.6 |
| 10 | Agarose (BF-160) | 4.0 g |
| 11 | Autoclave. | |
| 12 | Carbenicillin (40mg/ml - 1196) | 6.25 |
| 13 | TDZ (1mg/ml) | 1 ml |

**Table 4. Components of EJW 2 media.**

| **Order of Addition** | **Ingredient** | **Amount per Liter** |
|---|---|---|
| 1 | Gamborg's B5 Medium Phytotech G398 | 3.21 g |
| 2 | Sucrose (Phytotech S391) | 30 g |
| 3 | 2,4 D (1mg/ml) | 0.2 ml |
| 4 | MES | 2.0 g |
| 5 | Cleary's 3336 WP | 0.03 g |
| 6 | Stir until completely dissolved and solution is clear | |
| 7 | Add TC water to bring to this volume | 1000 ml |
| 8 | pH w/ KOH to | 5.6 |
| 9 | Agarose (BF-160) | 4.0 g |
| 10 | Autoclave. | |
| 11 | Carbenicillin (40mg/ml - 1196) | 6.25 |
| 12 | TDZ (1mg/ml) | 1 ml |

### Example 4: Particle Bombardment of Explants

### PDS1000 helium particle gun:

1. Gun components were sanitized for 1 min: using 70% EtOH for stop screens, rupture disks, and macrocarrier holders; and using isopropanol for carrier sheets.
2. Rupture disks were loaded (for example 1350 psi disks or disks in the range of approximately 650 - 2200 psi) into the rupture disk retaining cap and screwed into gas acceleration chamber.
3. A stop screen was placed on the brass adjustable nest.
4. 5 µl of helium gun prep was dispensed onto each carrier sheet for each bombardment. Carrier sheets were air dried before they were turned over and placed on top of a retaining screen on brass nest. The macrocarrier launch assembly was assembled, and placed directly under rupture disk. The gap distance between rupture disk and macrocarrier launch assembly was approximately 1 cm.
5. Precultured soy explants were positioned on a target plate medium #42 (TPM42) plate with meristems facing center and up and blasted.
6. The TPM42 medium was prepared by measuring 2 liters of distilled water into a 4 L beaker and adding 16 g of washed agar, which was then autoclaved for 25 minutes to bring the agar completely into solution. TPM42 may contain 8% carboxymethylcellulose (CMC) for low viscosity (or 2% carboxymethylcellulose (CMC) for high viscosity) and 0.4% of washed agar. The solution was cooled slightly and poured into a 4 L blender, and 320 g CMC (low viscosity) or 80 g CMC (high viscosity) were then added along with 2 L of water. The mixture was blended well and transferred to a 4 L plastic beaker, which was then autoclaved for 30 minutes, mixed and divided into four 1 L bottles. The TPM42 solution was then autoclaved for another 25 minutes and cooled to about 60 °C before being poured into plates. About 12 to 15 ml may be poured per 60 mm plate to make about 300 target plates, which may be stored at 4 °C or at -20 °C.

### ACCELL electric particle gun:

1. Bead prep was brought to room temperature and vortexed. A 0.5 Mil 3.2 cm² mylar sheet was placed onto a small plastic dish, optionally in a dehumidifier unit, and 320 µl of bead prep was placed onto the sheet. Each sheet was air dried.
2. Precultured soy explants were positioned on a TPM42 plate with meristems facing center and up.
3. A blank blast was done first due to inconsistencies in the energy of the first blast.
4. The target was placed over a retaining screen that was placed directly over the carrier sheet. Under a partial helium vacuum (13.5 in Hg), a 10 µL water droplet was vaporized by discharging the capacitor at 17.5 - 20 kV. The shock wave created by the vaporizing droplet propelled the sheet into the retaining screen, which stopped most of the mylar but allowed the gold beads to enter the soy explant meristems.
5. Between blasts, a drop of mineral oil was suspended between points and then removed to clean them. 10 µL of water was suspended between points as before. The arc chamber was covered with PVC block, a mylar sheet was placed on the square opening, and the screen hood was placed over the sheet and points. The screen was aligned over the sheet. The target dish was placed upside down over retaining screen such that meristems were oriented above it, and weight was placed on the dish. The apparatus was covered with bell jar and the vacuum was engaged. After 15 seconds, the vacuum read 13.5 in Hg, and the gun was discharged.

### Example 5: Culturing of Explants Following Particle Bombardment

1. Bombarded explants were surface plated onto EJW 1 media (or other pre-culturing media) overnight. In one example, plates were incubated at 28 °C with a 16/8 photoperiod.
2. For plastome transformations, explants were incubated the day after bombardment in 20% PEG4000 with 0.1 M Ca(NO₃)₂ for 1 hour, then rinsed.
3. The explants were surface plated or embedded onto 50-500 ppm spectinomycin- containing B5 media (LIMS 3485 with modified spectinomycin levels; Table 5) the next day, and kept at 28 °C with a 16/8 photoperiod. In one example, 250 ppm spectinomycin B5 media was used. The 24.5 g of B5 custom media mix included 3.21 g Gamborg's B5 medium, 20 g sucrose, and 1.29 g calcium gluconate.
4. Cultures were monitored for shoots/greening and subcultured as necessary. GFP was used as a marker in explants transformed with the GFP transgene.

**Table 5. Components of Lynx 3485 medium.**

| **Order of Addition** | **Ingredient** | **Amount per Liter** |
|---|---|---|
| 1 | B5 Custom Media Mix | 24.5 g |
| 2 | Cleary's 3336 WP (Carlin 10-032) | 0.03 g |
| 3 | Stir until completely mixed | |
| 4 | Ad TC water to bring to volume | 1000 ml |
| 6 | pH to | 5.6 |
| 7 | Agargel (Sigma A-3301) | 4g |
| 8 | Autoclave at 250 °F for 17 min | |
| 9 | Carbenicillin (40 mg/ml - 1195) | 5 ml |
| 10 | Timentin (100 mg/ml - 1585) | 1 ml |
| 11 | Cefotaxime (50 mg/ml - 1686) | 4 ml |
| 12 | Spectinomycin (50 mg/ml - 2387) | 3 ml |

### Example 6: Soy Plastid Transformation Events

Constructs used for soy plastid transformation of dry excised soybean embryo explants are shown schematically in FIG. 6. Table 6 summarizes results of several plastid transformation experiments using these dry excised explants. In these experiments, positive aadA-containing transformants were generally selected by culturing the explants in the presence of spectinomycin after bombardment. Post-bombardment culturing steps may further comprise sub-culturing of shoots on selection media with eventual rooting from the shoots.

**Table 6. Plastid transformation experiments using dry excised embryo explants from soybean.**

| **Trt** | **Explants bombarded** | **Construct** | **Genotype** | **Rehydration** | **Preculture** | **Rupture Disk** | **Selection** | **Greening explants subcultured** | **Greening explants GFP+** | **GFP+ greening explants with shoots / trifoliates ("Events")** | **GFP negative explants with shoots** |
|---|---|---|---|---|---|---|---|---|---|---|---|
| 1 | 352 | pMON286706 | A3555 | 10% sucrose | 2 day, B5 + 1 ppm TDZ +0.4% Agarose , light | 1550 psi | 1d rest on preculture, then 150 ppm spec B5 | 4 | 0 | 0 | 0 |
| 2 | 352 | pMON286706 | A3555 | 10% sucrose | 2 day, B5 + 1 ppm TDZ +0.4% Agarose , light | 1550 psi | 1d rest on preculture, then 150 ppm spec B5 | 7 | 0 | 0 | 0 |
| 2 | 197 | pMON286706 | A3555 | 10% sucrose | 1 day, B5 + 1 ppm TDZ +0.4% Agarose , light | 1350 psi | 1d rest on preculture, then 150 ppm spec B5 | 3 | 0 | 0 | 0 |
| 3 | 197 | pMON286706 | A3555 | 10% sucrose | 1 day, B5 + 2 ppm TDZ +0.4% Agarose , light | 1350 psi | 1d rest on preculture, then 150 ppm spec B5 | 1 | 0 | 0 | 0 |
| 3 | 192 | pMON286706 | A3555 | 10% sucrose | 2 day, B5 + 2 ppm TDZ +0.4% Agarose , light | 1350 psi | 1d rest on preculture, then 150 ppm spec B5 | 2 | 0 | 0 | 0 |
| 1 | 560 | pMON285270 | A3555 | 10% sucrose | 2 day, B5 + 2 ppm TDZ +0.4% Agarose , light | 1350 psi | 1d rest on preculture, then 150 ppm spec B5 | 18 | 5 | 1 | 0 |
| 1 | 304 | pMON285270 | A3555 | 10% sucrose | 1 day, B5 + 2 ppm TDZ +0.4% Agarose , light | 1350 psi | 1d rest on preculture, then 250 ppm spec B5 | 1 | 1 | 0 | 0 |
| 1 | 288 | pMON285270 | A3555 | 10% sucrose | 2 day, B5 + 2 ppm TDZ +0.4% Agarose , light | 1350 psi | 1d rest on preculture, 1 hr incubation in 20% PEG4000 + .1M Ca(NO3)2, then 250 ppm spec B5 | 4 | 4 | 0 | 0 |
| 1 | 592 | pMON285270 | A3555 | 10% sucrose | 1 day, B5 + 1 ppm TDZ +0.4% Agarose, light | 1350 psi | 1d rest on preculture, 1 hr incubation in 20% PEG4000 + .1M Ca(NO3)2, then 250 ppm spec B5 | 1 | 1 | 0 | 0 |
| 1 | 272 | pMON285270 | A3555 | 10% sucrose | 1 day, MS + 2 ppm TDZ + 0.4% Agarose , dark | 1350 psi | 1d rest on preculture, 1 hr incubation in 20% PEG4000 + .1M Ca(NO3)2, then 250 ppm spec B5 | 4 | 4 | 2 | 0 |
| 1 | 256 | pMON286706 | A3555 | 10% sucrose | 1 day, MS + 2 ppm TDZ + 0.4% Agarose , dark | 1350 psi | 1d rest on preculture, 1 hr incubation in 20% PEG4000 + .1M Ca(NO3)2, then 250 ppm spec B5 | 1 | 1 | 0 | 0 |
| 2 | 256 | pMON286706 | A3555 | 10% sucrose | 1 day, MS + 2 ppm TDZ + 0.4% Agarose , dark | 1350 psi | 1d rest on preculture, 1 hr incubation in 20% PEG4000 + .1M Ca(NO3)2, then 250 ppm spec B5 | 1 | 1 | 0 | 0 |
| 1 | 672 | pMON286706 | A3555 | 10% sucrose | 1 day, MS + 2 ppm TDZ + 0.4% Agarose , light | 1350 psi | 1d rest on preculture, 1 hr incubation in 20% PEG4000 + .1M Ca(NO3)2, then 250 ppm spec B5 | 3 | 0 | 0 | 1 |
| 1 | 608 | pMON286706 | A3555 | 10% sucrose | 2 day, MS + 2 ppm TDZ + 0.4% Agarose , light | 1350 psi | 1d rest on preculture, 1 hr incubation in 20% PEG4000 + .1M Ca(NO3)2, then 250 ppm spec B5 | 3 | 0 | 0 | 1 |
| 1 | 1280 | pMON285270 | A3555 | 10% sucrose | 1 day, MS + 1 ppm TDZ + 0.4% Agarose, light | 1350 psi | 1d rest on preculture, 1 hr incubation in 20% PEG4000 + .1M Ca(NO3)2, then 250 ppm spec B5 | 11 | 8 | 3 | 1 |
| 1 | 1280 | pMON285270 | A3555 | 10% sucrose | 2 day, MS + 1 ppm TDZ + 0.4% Agarose, light | 1350 psi | 1d rest on preculture, 1 hr incubation in 20% PEG4000 + .1M Ca(NO3)2, then 250 ppm spec B5 | 9 | 5 | 3 | 0 |
| 1 | 512 | pMON285270 | A3555 | 10% sucrose | 2 day, MS + 1 ppm TDZ + 0.4% Agarose, light | 1350 psi | 1d rest on preculture, 1 hr incubation in 20% PEG4000 + .1M Ca(NO3)2, then 250 ppm spec B5 | 10 | 3 | 0 | 0 |
| 2^{∗} | 512 | pMON285270 (2X DLR) | A3555 | 10% sucrose | 2 day, MS + 1 ppm TDZ + 0.4% Agarose, light | 1350 psi | 1d rest on preculture, 1 hr incubation in 20% PEG4000 + .1M Ca(NO3)2, then 250 ppm spec B5 | 13 | 3 | 2 | 0 |
| 1 | 928 | pMON285270 (2X DLR) | A3555 | 10% sucrose | 1 day, MS + 1 ppm TDZ + 0.4% Agarose, light | 1350 psi | 1d rest on preculture, 1 hr incubation in 20% PEG4000 + .1M Ca(NO3)2, then 250 ppm spec B5 | 2 | 2 | 0 | 0 |
| 1 | 464 | pMON286706 | A3555 | 10% sucrose | 2 day, MS + 1 ppm TDZ + 0.4% Agarose, light | 1350 psi | 1d rest on preculture, 1 hr incubation in 20% PEG4000 + .1M Ca(NO3)2, then 250 ppm spec B5 | 3 | 0 | 0 | 0 |
| 2 | 464 | pMON286706 (2X DLR) | A3555 | 10% sucrose | 2 day, MS + 1 ppm TDZ + 0.4% Agarose, light | 1350 psi | 1d rest on preculture, 1 hr incubation in 20% PEG4000 + .1M Ca(NO3)2, then 250 ppm spec B5 | 12 | 0 | 0 | 0 |
| 1 | 928 | pMON291978 | A3555 | 10% sucrose | 2 day, MS + 1 ppm TDZ + 0.4% Agarose, light | 1350 psi | 1d rest on preculture, 1 hr incubation in 20% PEG4000 + .1M Ca(NO3)2, then 250 ppm spec B5 | 1 | 1 | 1 | 0 |
| 1 | 416 | pMON291978 | A3555 | 10% sucrose | 1 day, MS + 1 ppm TDZ + 0.4% Agarose, light | 1350 psi | 1d rest on preculture, 1 hr incubation in 20% PEG4000 + .1M Ca(NO3)2, then 250 ppm spec B5 | 1 | 0 | 0 | 1 |
| 1 | 864 | pMON291978 | A3555 | 10% sucrose | 2 day, MS + 1 ppm TDZ + 0.4% Agarose, light | 1350 psi | 1d rest on preculture, 1 hr incubation in 20% PEG4000 + .1M Ca(NO3)2, then 250 ppm spec B5 | 1 | 0 | 0 | 1 |
| 1 | 960 | pMON291978 | AG3555 (RR2Y) | 10% sucrose | 1 day, MS + 1 ppm TDZ + 0.4% Agarose, light | 1350 psi | 1d rest on preculture, 1 hr incubation in 20% PEG4000 + .1M Ca(NO3)2, then 50 ppm spec B5 for 2weeks (surface plated); followed by 2 weeks on 150 ppm spec B5 (surface plated); followed by 250 ppm spec B5 | 1 | 0 | 0 | 1 |
| 1 | 736 | pMON291978 | AG3555 (RR2Y) | 10% sucrose | 1 day, MS + 1 ppm TDZ + 0.4% Agarose, light | 1350 psi | 3d rest on preculture, 1 hr incubation in 20% PEG4000 + .1M Ca(NO3)2, then 50 ppm spec B5 for 2weeks (surface plated); followed by 2 weeks on 150 ppm spec B5 (surface plated); followed by 250 ppm spec B5 | 6 | 0 | 0 | 0 |
| 1 | 800 | pMON291978 | AG3555 (RR2Y) | 10% sucrose | 1 day, MS + 1 ppm TDZ + 0.4% Agarose, light | 1350 psi | 1d rest on preculture, 1 hr incubation in 20% PEG4000 + .1M Ca(NO3)2, then 50 ppm spec B5 for 2weeks (surface plated); followed by 2 weeks on 150 ppm spec B5 (surface plated); followed by 250 ppm spec B5 | 3 | 0 | 0 | 0 |
| 1 | 352 | pMON291978 | AG3555 (RR2Y) | 10% sucrose | 2 day, MS + 1 ppm TDZ + 0.4% Agarose, light | 1350 psi | 2d rest on preculture, 1 hr incubation in 20% PEG4000 + .1M Ca(NO3)2, then 50 ppm spec B5 for 2weeks (surface plated); followed by 2 weeks on 150 ppm spec B5 (surface plated); followed by 250 ppm spec B5 | 5 | 0 | 0 | 1 |
| 2 | 352 | pMON285270 | AG3555 (RR2Y) | 10% sucrose | 2 day, MS + 1 ppm TDZ + 0.4% Agarose, light | 1350 psi | 2d rest on preculture, 1 hr incubation in 20% PEG4000 + .1M Ca(NO3)2, then 50 ppm spec B5 for 2weeks (surface plated); followed by 2 weeks on 150 ppm spec B5 (surface plated); followed by 250 ppm spec B5 | 5 | 3 | 1 | 0 |
| 1 | | pMON285270 | AG3555 (RR2Y) | 10% sucrose | 1 day, MS + 1 ppm TDZ + 0.4% Agarose, light | 1350 psi | 3d rest on preculture, 1 hr incubation in 20% PEG4000 + .1M Ca(NO3)2, then 50 ppm spec B5 for 2weeks (surface plated); followed by 2 weeks on 150 ppm spec B5 (surface plated); followed by 250 ppm spec B5 | 12 | 2 | 0 | 0 |
| 1 | 800 | pMON285270 | AG3555 (RR2Y) | 10% sucrose | 1 day, MS + 1 ppm TDZ + 0.4% Agarose, light | 1350 psi | 1d rest on preculture, 1 hr incubation in 20% PEG4000 + .1M Ca(NO3)2, then 50 ppm spec B5 for 2weeks (surface plated); followed by 2 weeks on 150 ppm spec B5 (surface plated); followed by 250 ppm spec B5 | 13 | 7 | 0 | 0 |
| 1 | 800 | pMON285270 | AG3555 (RR2Y) | 10% sucrose | 2 day, MS + 1 ppm TDZ + 0.4% Agarose, light | 1350 psi | 2d rest on preculture, 1 hr incubation in 20% PEG4000 + .1M Ca(NO3)2, then 50 ppm spec B5 for 2weeks (surface plated); followed by 2 weeks on 150 ppm spec B5 (surface plated); followed by 250 ppm spec B5 | 5 | 4 | 0 | 0 |
| 1 | 640 | pMON285270 | AG3555 (RR2Y) | 10% sucrose | 1 day, MS + 1 ppm TDZ + 0.4% Agarose, light | 1350 psi | 3d rest on preculture, 1 hr incubation in 20% PEG4000 + .1M Ca(NO3)2, then 50 ppm spec B5 for 2weeks (surface plated); followed by 2 weeks on 150 ppm spec B5 (surface plated); followed by 250 ppm spec B5 | 1 | 1 | 0 | 0 |
| 1 | 320 | pMON285270 | AG3555 (RR2Y) | 10% sucrose | 3 day, MS + 1 ppm TDZ + 0.4% Agarose, light | 1350 psi | 1d rest on preculture, 1 hr incubation in 20% PEG4000 + .1M Ca(NO3)2, then 50 ppm spec B5 for 2weeks (surface plated); followed by 250 ppm spec B5 | 3 | 3 | 0 | 0 |
| 1 | 640 | pMON285270 | AG3555 (RR2Y) | 10% sucrose | 2 day, MS + 1 ppm TDZ + 0.4% Agarose, light | 1350 psi | 2d rest on preculture, 1 hr incubation in 20% PEG4000 + .1M Ca(NO3)2, then 50 ppm spec B5 for 2weeks (surface plated); followed by 250 ppm spec B5 | 6 | 4 | 0 | 0 |
| 1 | 672 | pMON285270 | AG3555 (RR2Y) | 10% sucrose | 1 day, MS + 1 ppm TDZ + 0.4% Agarose, light | 1350 psi | 3d rest on preculture, 1 hr incubation in 20% PEG4000 + .1M Ca(NO3)2, then 50 ppm spec B5 for 2weeks (surface plated); followed by 250 ppm spec B5 | 4 | 1 | 0 | 0 |
| 1 | 704 | pMON286706 | AG3555 (RR2Y) | 20% PEG4000 | 1 day, MS + 1 ppm TDZ + 0.4% Agarose, light | 1350 psi | 1d rest on preculture, 1 hr incubation in 20% PEG4000 + .1M Ca(NO3)2, then 50 ppm spec B5 for 2weeks (surface plated); followed by 250 ppm spec B5 | 2 | 0 | 0 | 0 |
| 1 | 736 | pMON286706 | AG3555 (RR2Y) | 20% PEG4000 | 1 day, MS + 1 ppm TDZ + 0.4% Agarose, light | 2000 psi | 1d rest on preculture, 1 hr incubation in 20% PEG4000 + .1M Ca(NO3)2, then 50 ppm spec B5 for 2weeks (surface plated); followed by 250 ppm spec B5 | 1 | 0 | 0 | 0 |
| 1 | 384 | pMON285270 | AG3555 (RR2Y) | 20% PEG4000 | 1 day, MS + 1 ppm TDZ + 0.4% Agarose, light | 2000 psi | 1d rest on preculture, 1 hr incubation in 20% PEG4000 + .1M Ca(NO3)2, then 50 ppm spec B5 for 2weeks (surface plated); followed by 500 ppm spec B5 | 1 | 1 | 0 | 0 |
| 1 | 416 | pMON285270 | AG3555 (RR2Y) | 20% PEG4000 | 2 day, MS + 1 ppm TDZ + 0.4% Agarose, light | 2000 psi | 2d rest on preculture, 1 hr incubation in 20% PEG4000 + .1M Ca(NO3)2, then 50 ppm spec B5 for 2weeks (surface plated); followed by 500 ppm spec B5 | 1 | 1 | 0 | 0 |

| | | | | | | | | | | | |
|---|---|---|---|---|---|---|---|---|---|---|---|
| ^{∗} 2 rooting shoots generated from event 6. | | | | | | | | | | | |

### Example 7: Molecular Analysis of Soy Plastid Transformants

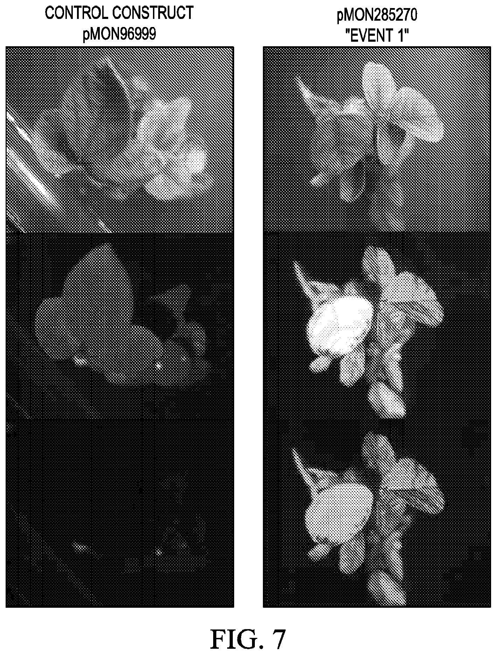

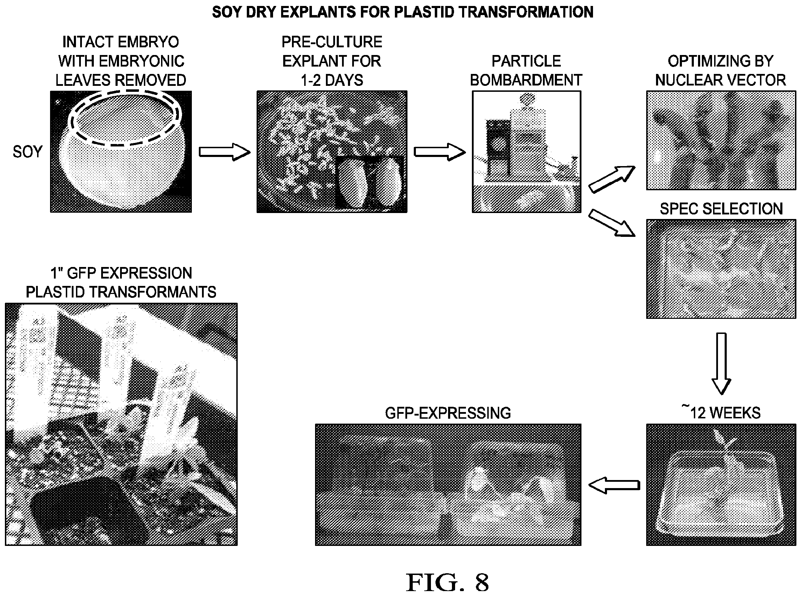

A GFP positive shoot was recovered with spectinomycin selection from soybean explants transformed with the pMON285270 construct and designated "Event 1". Event 1 was generated using the pMON285270 construct and showed strong GFP positive expression in shoots compared with a control GFP negative event. Plants growing from the GFP-positive shoot comprising "Event 1" exhibited strong GFP expression. As shown for example in FIG. 7, Ro plant shoots comprising the Event 1 transgene displayed robust and ubiquitous GFP expression (bottom right image), whereas control plants bombarded with a nuclear expression vector, pMON96999 (for a plasmid map of pMON96999, see, e.g., U.S. Patent No. 8,466,345), had no GFP fluorescence (bottom left image) when evaluated under a "GFP3" setting with LEICA software using excitation filter 470/40 nm (450-490 nm) and barrier filter 525/50 nm (500-550 nm). The two middle images in FIG.7 were used as controls under a "GFP2" setting generated using excitation filter 480/40 nm (460-500 nm) and barrier filter 510 LP. The GFP2 LEICA setting includes emission light above 510 nm, whereas the GFP3 setting excludes emission light above 550 nm. Since chlorophyll can be excited around the same light wavelength range as GFP and emit light over 600 nm, the GFP2 setting includes chlorophyll fluorescence, but the GFP3 setting should generally be limited to the GFP fluorescence. The GFP2 filter set may also be used to distinguish living versus dead or necrotic tissue. A thin section was taken from Event 1 plant shoot tissues and examined under confocal microscope to confirm plastid GFP expression (data not shown). An overview of a process used to make transplastomic (GFP-positive) Ro plants by direct plastid transformation of an embryo explant meristem (without any callus phase) according to embodiments of the present invention is shown in FIG. 8.

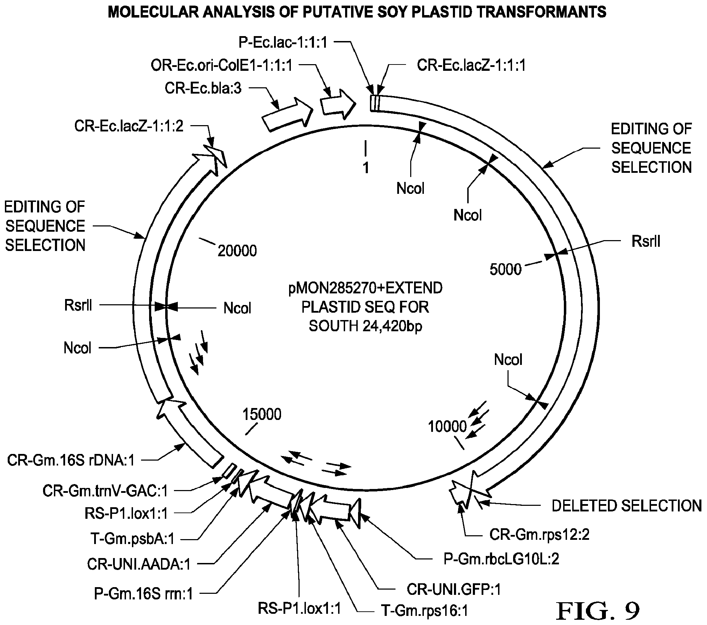

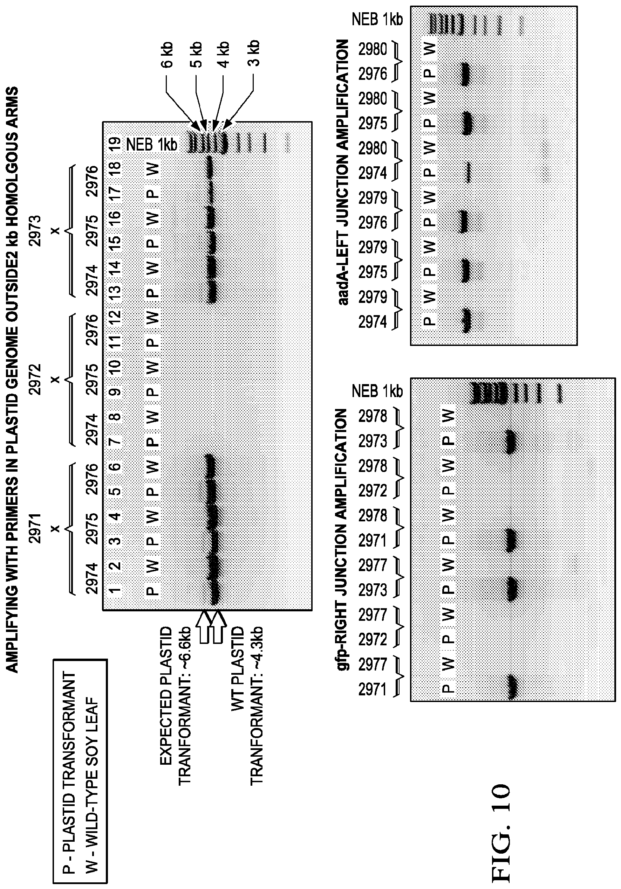

Plastomic transformation was also confirmed by PCR using a leaf sample (FIGS. 9 and 10). The Event 1 transformant showed a larger ~6.6 kb band expected with plastid DNA primers flanking the insertion in addition to a smaller ~4.3 kb band expected for wild-type plastid DNA without the insertion. Primer pairs for amplifying fragments at the gfp-right junction (Xd2971 (SEQ ID NO: 15) / Xd2977 (SEQ ID NO: 21); Xd2973 (SEQ ID NO: 17) / Xd2977 (SEQ ID
NO: 21); Xd2973 (SEQ ID NO: 17) / Xd2978 (SEQ ID NO: 22)) and *aadA*-left junction (Xd2974 (SEQ ID NO: 18) / Xd2979 (SEQ ID NO: 23); Xd2975 (SEQ ID NO: 19) / Xd2979 (SEQ ID NO: 23); Xd2976 (SEQ ID NO: 20) / Xd2979 (SEQ ID NO: 23); Xd2974 (SEQ ID NO: 18) / Xd2980 (SEQ ID NO: 24); Xd2976 (SEQ ID NO: 20) / Xd2980 (SEQ ID NO: 24)) also produced the expected plastid transformation bands (FIGS. 9 and 10). However, a primer flanking *rps12* of the insertion (Xd2972) did not produce an amplification product in either wild- type or with the putative plastid transformant samples, which may be due to poor primer synthesis or non-specificity.

Several additional GFP positive events were recovered from explants transformed with the pMON285270 construct. Seven of these putative soy plastid transformants produced shoots under spectinomycin selection and were evaluated for plastid transformation using PCR. As shown in FIG. 11, several positive transformants were identified from these putative transformation events that exhibited GFP expression and produced the ~6.6 kb amplification product expected for a targeted plastid transformation event by PCR using primers Xd2971 (SEQ ID NO: 15) and Xd2976 (SEQ ID NO: 20). These same transformants also produced an expected ~6.4 kb amplification product by PCR using primers Xd2973 (SEQ ID NO: 17) and Xd2974 (SEQ ID NO: 18), confirming successful plastid transformation.

These plastid transformants were further analyzed for the presence of a *gƒp-aadA* junction fragment by amplifying samples using primers Xd2599 (SEQ ID NO: 25; *gfpuvm* forward) and Xd2606 (SEQ ID NO: 26; *aadA* reverse). As expected, these events produced using pMON285270 yielded a 791 bp *gƒp-aadA* junction fragment product, while events produced using pMON291978 yielded a 886 bp *gƒp-aadA* junction fragment product (FIG. 11).

Samples 1, 2, and 3 from transformants using pMON285270, which exhibited GFP expression and generated amplification products by PCR indicative of plastid transformation, were designated Event 4, Event 5, and Event 8, respectively. However, samples 4-7 did not produce the expected PCR bands and were GFP negative (Table 7). Although sample 5 may have produced a *gƒp-aadA* junction band in FIG. 11, a larger ~6.4 or ~6.6 PCR product expected for the insert was not observed.

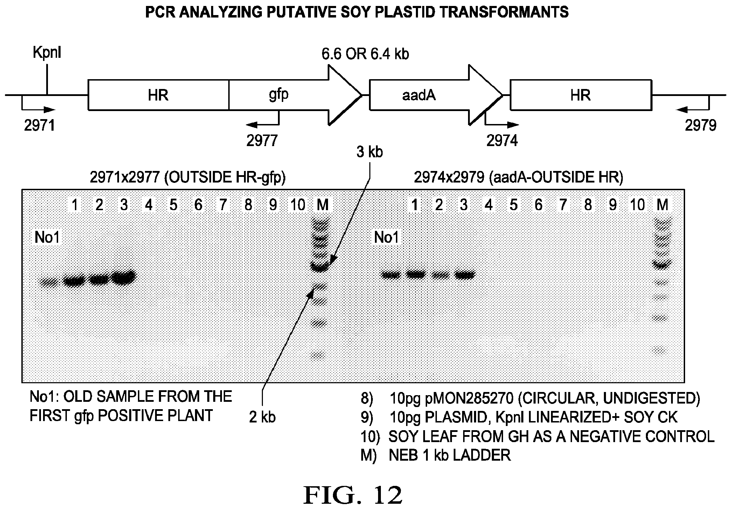

These seven putative soy plastid transformation events were further analyzed for the presence of a *gfp*-HR junction fragment using primer pair Xd2971 (SEQ ID NO: 15) and Xd2977 (SEQ ID NO: 21), and the presence of an *aadA*-HR junction fragment using primer pair Xd2974 (SEQ ID NO: 18) and Xd2979 (SEQ ID NO: 23), as shown in FIG. 12. Samples 1, 2, and 3 (designated Event 4, Event 5, and Event 8, respectively) each comprised both *gfp*-HR junction fragment and an *aadA*-HR junction fragment, whereas samples 4-7 did not. These results further confirm that Events 4, 5 and 8 represent successful transformations with exogenous DNA insertions at the target site in the plastid genome. Although additional shoots and/or rooting explants were obtained (see samples 4-7), they were GFP-negative and did not produce the expected PCR amplification products by PCR, perhaps as a result of truncated plastid events, genomic insertions, and/or promoter trapping of the selectable marker gene.

**Table 7. Putative plastid transformants.**

| **Sample** | **Designation** | **GFP** | **Construct** | **Status** | **Conditions** |
|---|---|---|---|---|---|
| 1 | "Event 4" | positive | pMON285270 | rooting shoot | 1 d MS + 1 ppmTDZ preculture; 1350 psi |
| 2 | "Event 5" | positive | pMON285270 | rooting shoot | 1 d MS + 1 ppmTDZ preculture; 1350 psi |
| 3 | "Event 8" | positive | pMON285270 | shoot | 2 d MS + 1 ppm TDZ preculture; 1350 psi |
| 4 | none | not detected | pMON285270 | rooting shoot | 1 d MS + 1 ppm TDZ preculture; 1350 psi |
| 5 | none | not detected | pMON291978 | shoot | 1 d MS + 1 ppm TDZ preculture; 1350 psi |
| 6 | none | not detected | pMON291978 | shoot from rooting explant | 2 d MS + 1 ppm TDZ preculture; 1350 psi |
| 7 | none | not detected | pMON291978 | shoot | 2 d MS + 1 ppm TDZ preculture; 1350 psi |

The presence of GFP-positive chloroplasts in mesophyll leaf cells was also confirmed by confocal microscopy of greening tissues from plastid transformants. Numbers of GFP-positive plants obtained in several rounds of particle bombardment along with various treatment conditions are shown in Table 8.

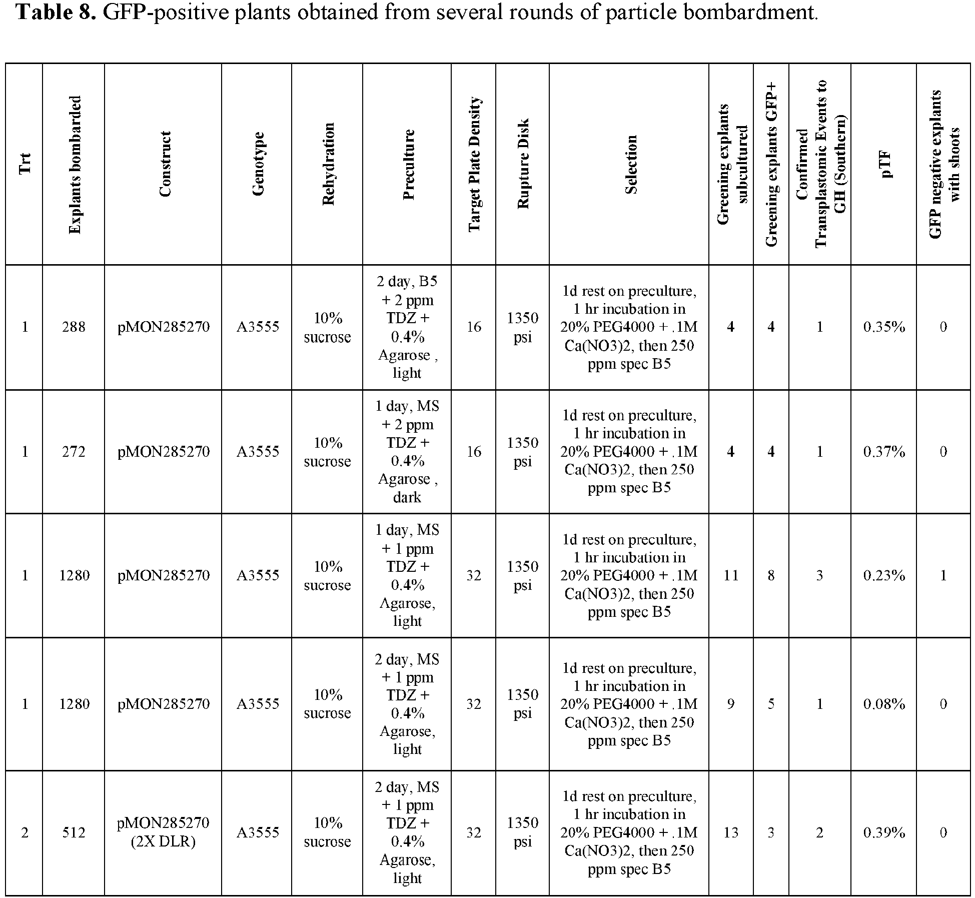

### Example 8: Increasing Homoplastomy of Soy Plastid Transformants by Selection

The paternal explant of Event 1 was subcultured onto fresh 250 ppm spec B5 47 days after blasting. A primary shoot was harvested on 150 ppm spec in bean rooting medium (BRM) (LIMS 4055; Table 9), and the parental explant shoot was subcultured again onto fresh 250 ppm spec 66 days after intial blasting. The parental explant was freshly cut to remove necrotic tisuse on the hypcotyl, and subcultured again 78 days after initial blasting onto fresh 250 ppm spec B5. The resulting rooted explant was sent to the greenhouse 116 days after initial blasting, together with rooted shoots from Events 4 and 5 (Table 10). The events shown in Table 10 were imaged under GFP blue light with yellow filter 138 days after initial blasting, and persistence of the ubiquitous GFP expression was observed throughout the plant.

**Table 9. Components of LIMS 4055 (BRM).**

| **Order of Addition** | **Ingredient** | **Amount per Liter** |
|---|---|---|
| 1 | MS Basal Salts | 2.15 g |
| 2 | Myo-inositol | 0.1 g |
| 3 | Sucrose | 30 g |
| 4 | MS SSC Vitamin (500x) | 2 ml |
| 6 | Cysteine (10 mg/ml) | 10 ml |
| 7 | Stir until completely dissolved and solution is clear | |
| 9 | Add TC water to volume | 1000 ml |
| 10 | pH w/ KOH to | 5.8 |
| 11 | Agar, Bacto | 8 g |
| 12 | Autoclave. | |
| 13 | Spectinomycin (50 mg/ml) | 3 ml |
| 14 | IAA (0.033 mg/ml) | 10 ml |
| 15 | Timentin (100 mg/ml) | 1 ml |

**Table 10. Evaluation of Events 1, 4, and 5 for persistance of GFP signal.**

| **Event Name** | **Construct** | **Designation** | **Subcultured (days post blast)** | **Shoot Harvest (days post blast)** | **Subcultured (days post blast)** |
|---|---|---|---|---|---|
| GM_A21178618 | pMON285270 | Event 4 rooted shoot | 48 days | 79 days | |
| GM_A21178619 | pMON285270 | Event 5 rooted shoot | 48 days | 79 days | |
| GM A21178620 | pMON285270 | Event 1 rooted explant (subcultured) | 47 days | 67 days | 79 days |

In separate experiments, explants were bombarded with particles coated at twice the exogenous DNA loading rate (i.e., 2.4 µ g DNA / mg gold beads instead of the standard prep of 1.2 µg DNA / mg beads used in the examples above). Two rooted shoots were recovered with subculturing under spectinomycin selection from an explant and designated "Event 6." These two Event 6 plantlets were sent to the greenhouse on day 121 after initial blasting, and both exhibited strong and ubiquitous GFP activity (Table 11). Persistence of GFP expression was observed in both of these Event 6 plants (compared to genomic control) when exposing these R₀ plants to overhead blue lights (Orbitech) and visualized using a yellow barrier filter over a digital camera.

**Table 11. Evaluation of Event 6 for persistance of GFP signal.**

| **Event Name** | **Exp-Trt#** | **Construct** | **Designation** | **Subcultured (days post blast)** | **Shoot Harvest (days post blast)** |
|---|---|---|---|---|---|
| GM_A21182160 | 501084-1 | pMON285270 | Event 6 rooted shoot | 44 days | 75 days |
| GM_A21182161 | 501084-1 | pMON285270 | Event 6 rooted shoot | 44 days | 75 days |

The five R₀ transplastomic plants summarized in Tables 10 and 11 (i.e., corresponding to Events 1, 4, 5 and 6, which included two plants for Event 6) were analyzed under a GFP lighting system with yellow barrier filter at later time points (e.g., out to day 150 after blasting and beyond), and persistence of the uniform and ubiquitous GFP reporter expression was observed even after removal of the selection pressure and potting of the plants into soil. This demonstrates that the plastid transformation events in these R₀ plants are stable over the life of these plants since continued use of the selection agent was not required to maintain widespread GFP expression throughout these transplastomic plants.

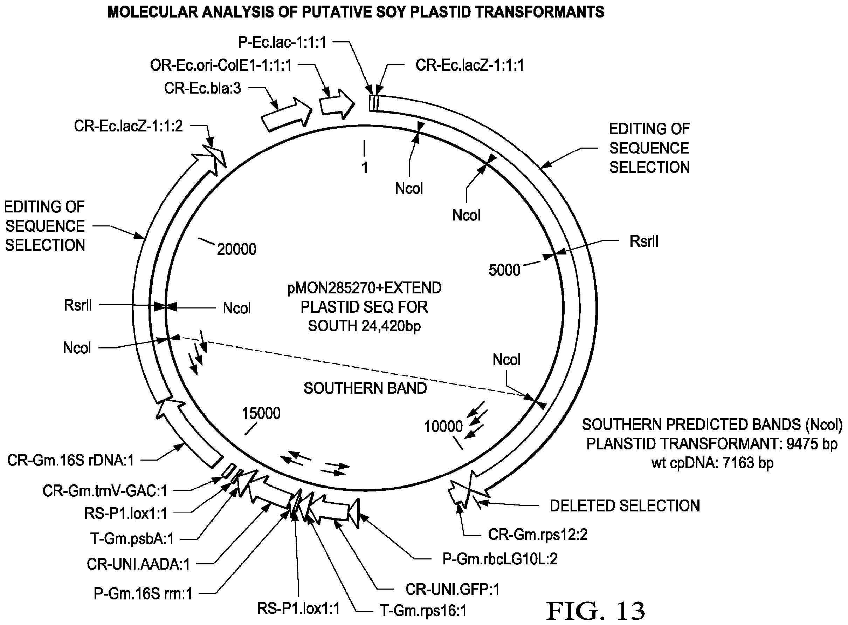

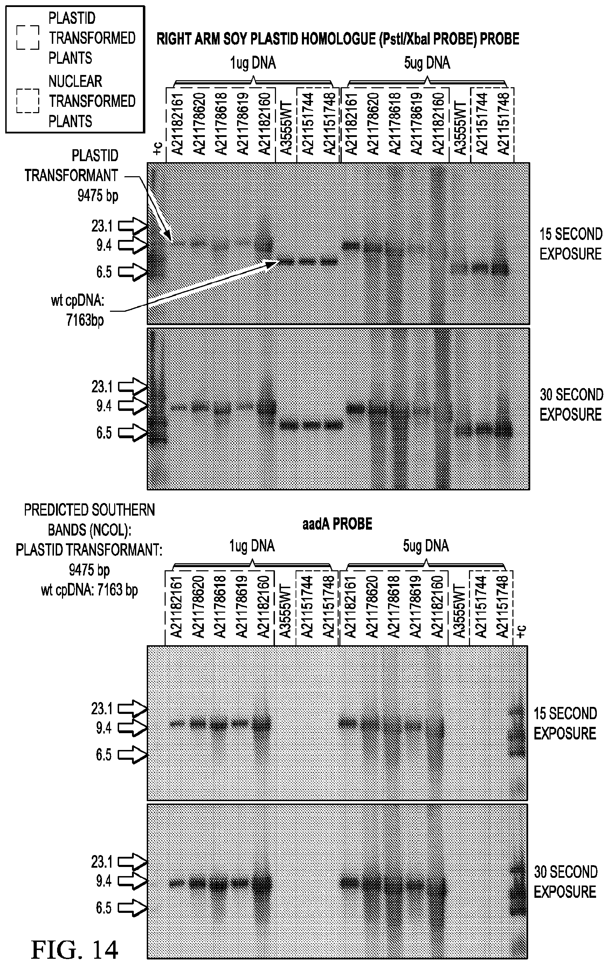

The transplastomic character of these five R₀ plants was further confirmed by Southern analysis following an NcoI digestion. As shown in FIG. 14, plastid transformants are expected to exhibit a 9,475 bp band when hybridized and labeled with a PstI/XbaI probe, while wild type plants are expected to exhibit a 7,163 bp band of the DNA prep (see also FIG. 13 showing a graphical representation of the expected transgenic soy plastid DNA with restriction sites). Indeed, each of the Events summarized in Tables 10-12 shows the 9,475 bp band indicating successful plastid transformation at the targeted plastid DNA locus. Southern analysis was also performed by labeling the NcoI digested blot with a probe for the *aadA* transgene. In these experiments, only lanes corresponding to the transplastomic Events produced the labeled band of expected size (FIG. 14), further confirming successful plastid transformation with these Events.

Additional rooted explants were also obtained that were GFP-positive (Table 12), and GFP signal in roots was also been detected (presumably in leucoplasts).

**Table 12. Additional GFP-positive rooted explants.**

| **Event Name** | **Construct** | **Designation** | **Subcultured (days post blast)** | **Shoot Harvest (days post blast)** | **Subcultured* (days post blast)** | **Subcultured* (days post blast)** |
|---|---|---|---|---|---|---|
| GM_A21195637 | pMON285270 | rooted explant | 63 days | | 97 days | 150 spec BRM 120 days |
| GM A21195638 (dark preculture) | pMON285270 | rooted explant from "event 7" | 57 days | | 110 days | 150 spec BRM 127 days |
| GM A21195639 | pMON285270 | rooted explant | 48 days | 72 days | 150 spec BRM 118 days | |

| | | | | | | |
|---|---|---|---|---|---|---|
| ^{∗}Explant given fresh cut. | | | | | | |

### Example 9: Confirmation of Transformed Plastid Inheritance

Reporter GFP expression was also observed in immature pods and R1 seed (with and without maternal seed coat) from the Event 4 (GM_A21178618) transplastomic R₀ plant, confirming plastid inheritance to progeny (FIG. 15). The top left set of images in FIG. 15 show GFP expression in transgenic immature pods (see lower left image) in comparison to a GFP-negative control plant (bombarded with a nuclear transformation vector, pMON96999); the top right set of images in FIG. 15 show GFP expression in transgenic immature pods and seeds (see lower left plate; pods bisected to show seeds); the bottom left set of images in FIG. 15 show GFP expression in transgenic intact seeds (see lower left image); and the bottom right set of images in FIG. 15 show GFP expression in transgenic seeds with the seed coat removed (see lower left image). All 13 R1 seeds examined from the GM_A21178618 R₀ plant were GFP positive, which would be expected for progeny of a R₀ plant homoplastomic for a transplastomic event (or at least from a nearly homoplastomic or ubiquitous R₀ event).

### Example 10: Inheritance and Confirmation of Effective Homoplasmy in R1 Generation

R1 seed from eight soy plastid transformation events were germinated in greenhouse conditions without selection and plants were imaged for the GFP marker. All R1 soybean plantlets from each of the lines were positive, with no obvious sectoring. Some of these soy plants were then transplanted to further obtain R2 generation seed. Plant height and GFP activity were measured at various times was imaged. Again, uniform GFP expression was found throughout these R2 plants in the absence of selection. The presence of GFP in chloroplasts was confirmed by examining R1 leaf cells under laser confocal microscopy. GFP in chloroplasts was visible at the periphery of cells surrounding the central vacuole. By contrast, the GFP signal in the nuclear transformed control line was visible in the cytosol of cells.

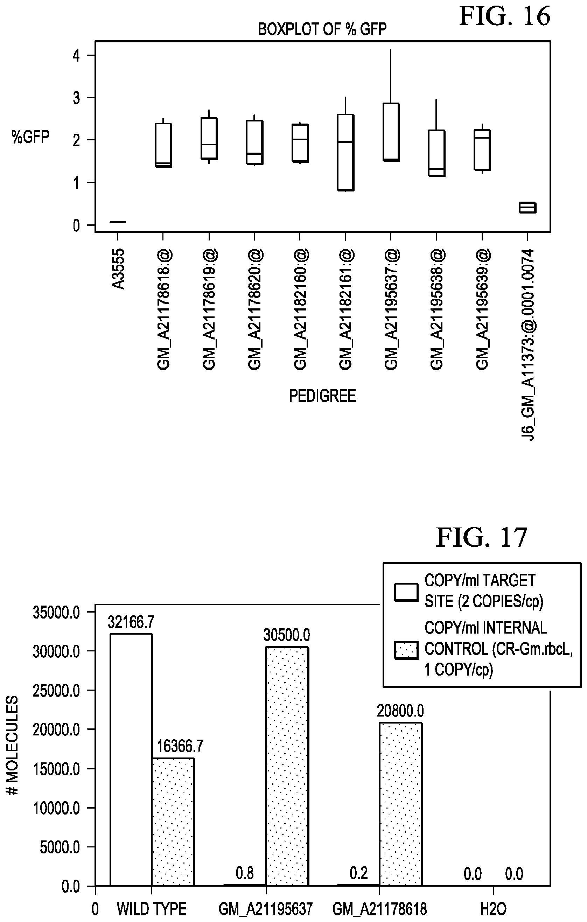

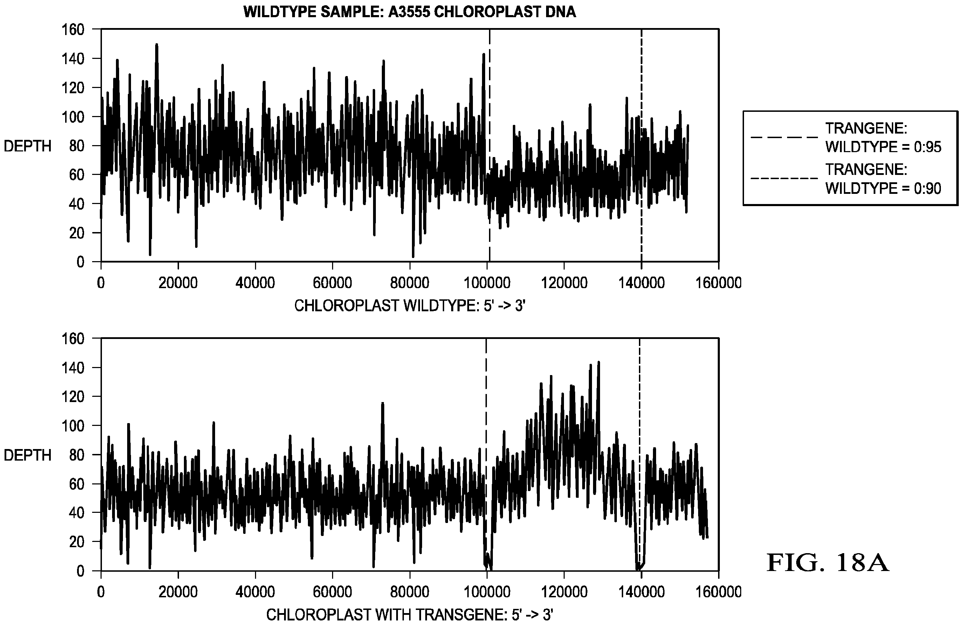

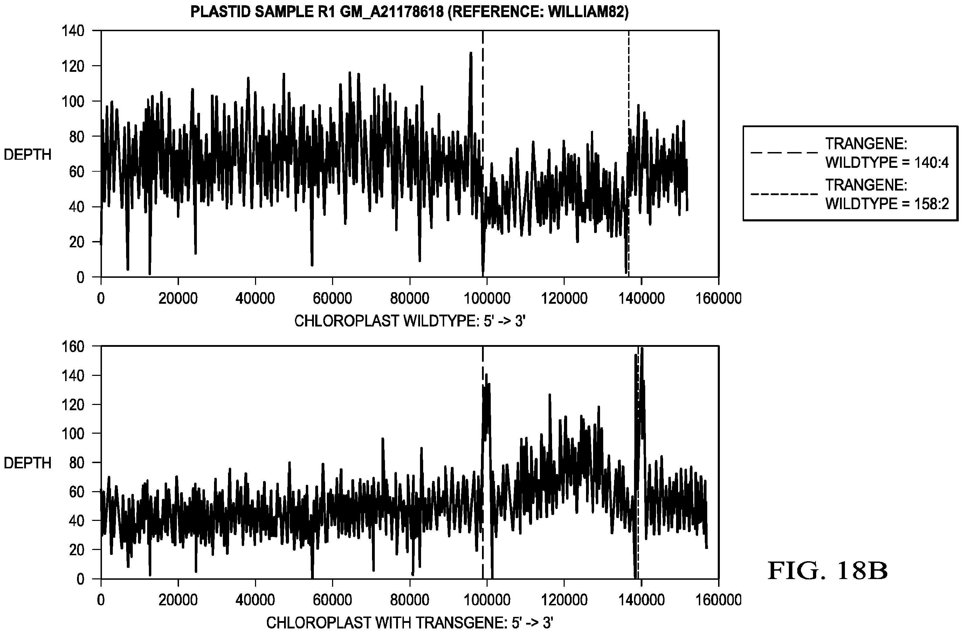

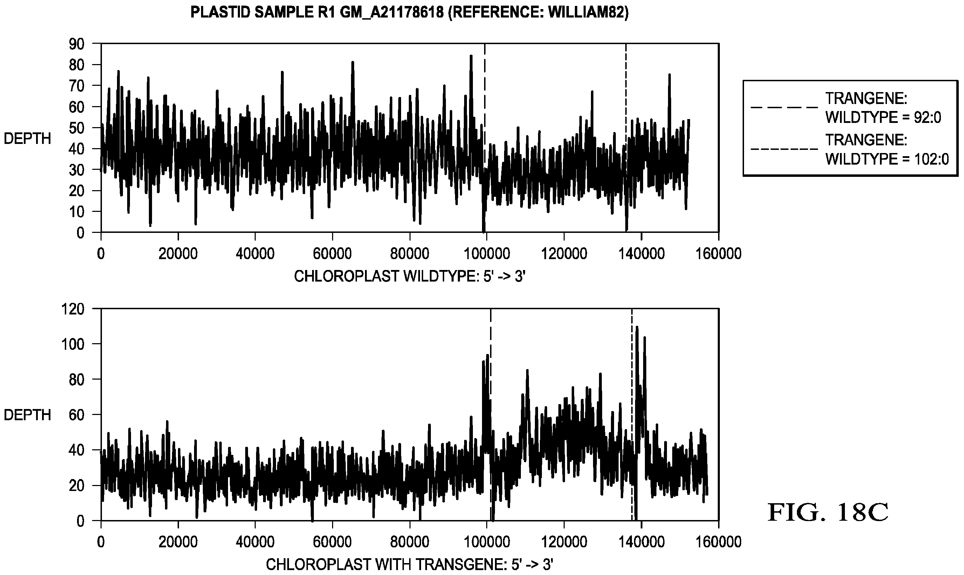

R1 soybean plants were further sampled to estimate GFP protein expression levels using a BioVision kit (BioVision, Inc. Milpitas, CA), and were calculated as a percentage of total soluble protein using the Bradford assay. Quantification of GFP in transplastomic events compared with nuclear GFP and wild type control is shown in FIG. 16. R1 plants were also sampled and purified chloroplast DNA prepared from these samples was subjected to Southern analysis, which again confirmed their transplastomic character with an expected 9.4 kb band. The large subunit of RuBisCO was also used as a 1-copy internal control in these experiments. Samples from both of the transplastomic lines had the expected disruption at the targeted integration site (i.e., a probe-labeled 9.4 kb band) without detection of the 6.5 kb wild type band, which was present in the control samples (data not shown), thus indicating a state of effective homoplastomy in these transgenic lines. Leaf samples from six different R1 plants were pooled for each of two transplastomic lines, and DNA preparations (isolated according to the Shi *et al.* 2012 protocol) from these samples as well as wild type leaf samples were analyzed by digital Droplet Digital^{™} PCR (ddPCR^{™}; Bio-Rad^{®}) (FIG. 17). Loss of the expected wild type PCR product was observed in samples from the transplastomic events. These samples were also sent for PacBio^{®} sequencing analysis with Illumina^{®} finishing (FIGS. 18A-18C), which further demonstrated the homoplastomic (or nearly homoplastomic) state of these R1 samples by the almost exclusive presence of the transplastomic insertion sequence relative to the wild type sequence. William 82 wild type soybean line was used as a reference for the transgenic samples.
As further evidence that these R1 plants were uniformly transplastomic, R1 seed was germinated in the presence of 150 ppm spectinomycin along with wild-type control seed, which was sufficient to bleach plastids in the control tissue, while no sectoring or segregation was observed in the R1 plants.

### Example 11: Crossing Results and Inheritance in R2 Generation

To demonstrate lack of paternal inheritance of the transplastomic events, pollen from four R1 soybean plants (two plants for each of the two transplastomic event lines) was crossed onto wild-type emasculated flowers. Pollen from a homozygous GUS nuclear transgene line was also crossed as a positive control. The resulting seed was sanitized in 10% Clorox for about 10 minutes, rinsed, and germinated on either B5 media without selection or on 150 ppm spectinomycin B5. Phenotypes and GFP activity were evaluated 2 weeks later. As expected, pollen from nuclear GUS control plants resulted in F1 spectinomycin resistant (and GUS+) hemizygotes, while pollen from plastid GFP plants resulted in spectinomycin-sensitive and GFP- negative F1 plants. The expected chloroplast expression was observed in these GFP-positive plants using laser confocal microscopic imaging. These results are shown in Table 13.

**Table 13. F1 Crossing Results and Inheritance in R2 Generation.**

| **Line** | **Seeds germinated on B5** | **Seeds germinated on 150 spec B5** | **GFP** |
|---|---|---|---|
| Wild type Control | 12/12 | 0/9 (all bleached) | 0/21 |
| F1 Nuclear GUS (cross) GM_A21151686@0002 pollen | 24/24 | 24/24 | 0/48 GFP |
| R2 Plastid (self) GM_A21178618@0017 | 12/12 | 12/12 | 24/24 |
| R2 Plastid (self) GM_A21195637@0023 | 12/12 | 12/12 | 24/24 |
| F1 Plastid (cross) GM_A21178618@0005 pollen | 26/26 | 0/28 (all bleached) | 0/54 |
| F1 Plastid (cross) GM_A21178618@0006 pollen | 24/24 | 0/26 (all bleached) | 0/50 |
| F1 Plastid (cross) GM_A21195637@0011 pollen | 22/22 | 0/22 (all bleached) | 0/44 |
| F1 Plastid (cross) GM_A21195637@0012 pollen | 24/24 | 0/24 (all bleached) | 0/48 |

### Example 12: Additional GFP Transplastomic Soy Events Generated from Alternate Selection and Preculturing Medias

A confirmed transplastomic plant using the pMON285270 GFP construct was obtained using 150 ppm spectinomycin selection, rather than 250 ppm spectinomycin as used in prior experiments. This plant was also moved off selection only 65 days after its bombardment. Shoots obtained using this altered protocol was rooted on spectinomycin as a screen to determine if this shortened selection period was sufficient to generate transplastomic soybean plants. Plants containing this event displayed uniform GFP expression similar to transplastomic plants generated with the heightened selection protocol.

Transplastomic events from the control GFP construct were also obtained by replacing the TDZ in the preculturing and rest media (LIMS 4859; Table 14) with 5 ppm kinetin. These events were also generated using 150 ppm spectinomycin selection without the use of the 20% PEG2000 + 0.1 M Ca(NO₃)₂ rinse post bombardment. Individual plant events were derived from the same explant (first being a rooted shoot cut from the explants, second being a plant derived from the remaining explant that rooted). These events displayed uniform GFP expression and confocal localization of the GFP signal to chloroplasts in lead mesophyll cells. Southern analysis of these R₀ plants appears in FIG. 17 alongside Southern analysis of the R1 events described above.

**Table 14. Components of LIMS 4859 medium.**

| **Order of Addition** | **Ingredients** | **Amount per Liter** |
|---|---|---|
| 1 | TC Water | 800 mL |
| 2 | Agarose (VWR 0710) | 4 g |
| 3 | MS Basal Salts (Phytotech M524) | 4.33 g |
| 4 | Sucrose (Phytotech S391) | 30 g |
| 5 | 2,4-D (1 mg/mL) (Phytotech D295) | 0.2 mL |
| 6 | MES Hydrate (Sigma M8250) | 2 g |
| 7 | Clearys 3336WP | 0.03 g |
| 8 | Bring to volume (1L) with TC Water | -- |
| 9 | pH with KOH | 5.6 |
| 10 | Autoclave | -- |
| | Add the following fertile sterilized ingredients: | |
| 11 | Thidiazuron ( 1 mg/mL) | 1 mL |
| 12 | Carbenicillin (40 mg/mL) | 6.25 mL |

A summary of transplastomic soybean event generation protocols using the pMON285270 construct are given in Tables 15 and 16 below, with totals provided in Table 17.

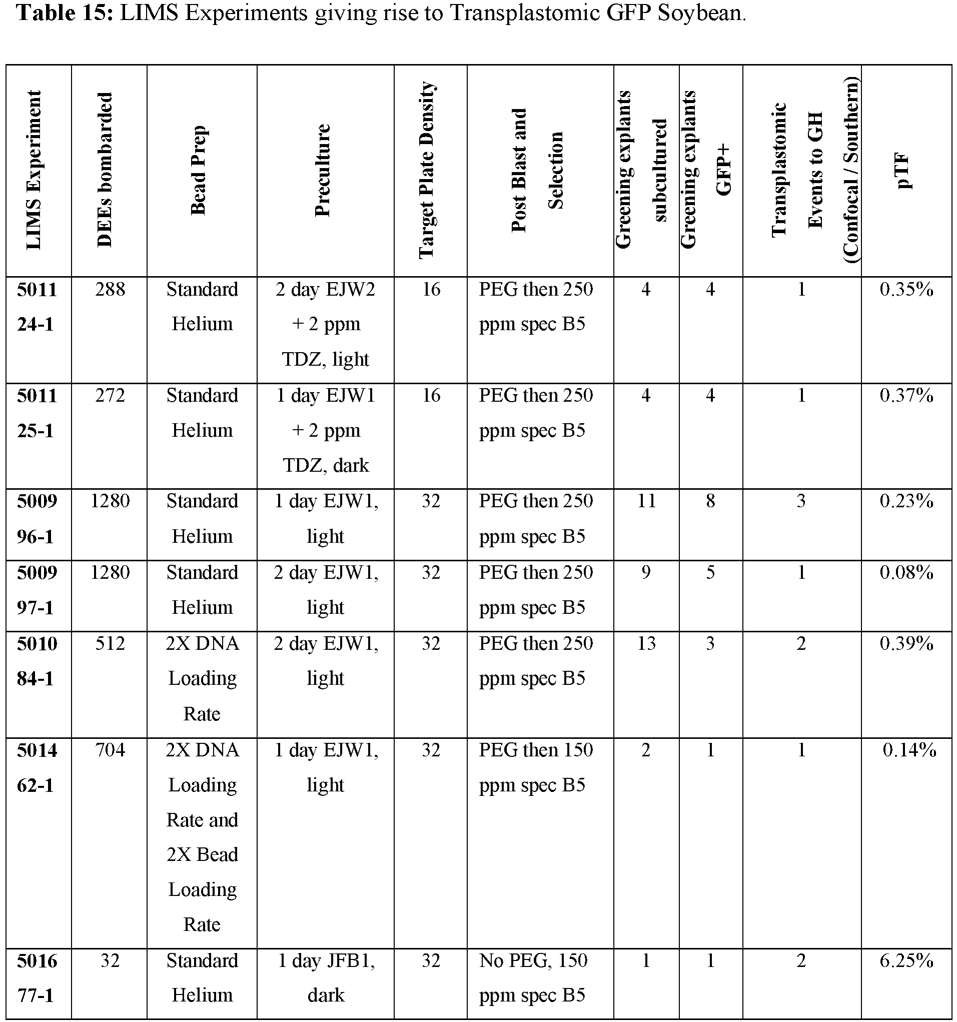

**Table 16: Transplastomic GFP Soybean Events with Corresponding Experiments and Subculturing Schemes (Days measured from date of explant bombardment).**

| **Event Name** | **Exp-Trt#** | **Designation** | **Subculture** | **Days to Greenhouse** |
|---|---|---|---|---|
| **GM_A21178618** | 500996-1 | Rooted shoot | Moved to fresh 250 ppm spec B5 at day 48; shoot harvested onto 150 ppm spec BRM at day 79 | 117 days |
| **GM_A21178619** | 500996-1 | Rooted shoot | Moved to fresh 250 ppm spec B5 at day 48; shoot harvested onto 150 ppm spec BRM at day 78 | 117 days |
| **GM_A21178620** | 500997-1 | Rooted explant (from "event 1") | Moved to fresh 250 ppm spec B5 at day 47; shoot harvested onto 150 ppm spec BRM at day 66 and explant retained; explant subcultured onto fresh 250 ppm spec B5 at day 78 | 116 days |
| **GM_A21182160** | 501084-1 | Rooted shoot | Moved to fresh 250 ppm spec B5 at day 44; shoot harvested onto 150 ppm spec BRM at day 75 | 121 days |
| **GM_A21182161** | 501084-1 | Rooted shoot (from same explant as GM_A21182160) | Moved to fresh 250 ppm spec B5 at day 44; shoot harvested on 150 ppm spec BRM at day 75 | 121 days |
| **GM_A21195637** | 501124-1 | Rooted explant | Moved to fresh 250 ppm spec B5 at day 63; explant given fresh cut to remove necrotic tissue and moved to fresh 250 ppm spec B5 at day 97; explant given fresh cut to remove necrotic tissue and moved to 150 ppm spec BRM at day 120 | 162 days |
| **GM_A21195638** | 501125-1 | Rooted explant | Moved to fresh 250 ppm spec B5 at day 57; explant given fresh cut to remove necrotic tissue and moved to fresh 250 ppm spec B5 at day 110; explant given fresh cut to remove necrotic tissue and moved to 150 ppm spec BRM at day 127 | 156 days |
| **GM_A21195639** | 500996-1 | Rooted explant | Moved to fresh 250 ppm spec B5 at day 48; shoot harvested onto 150 ppm spec BRM at day 72 and explant retained; explant given fresh cut to remove necrotic tissue and moved to 150 ppm spec BRM at day 118 | 147 days |
| **GM_A21293034** | 501462-1 | Rooted shoot | Moved to fresh 150 ppm spec B5 at day 50; moved to B5 without selection at day 65; shoot harvested on 150 ppm spec BRM at day 79 | 114 days |
| **GM_A21320569** | 501667-1 | Rooted shoot | Moved to fresh 150 ppm spec B5 at day 30; moved to fresh 150 ppm spec B5 at day 37; moved to 50 ppm spec B5 at day 52; shoot harvested on 150 ppm spec BRM at day 59 | 78 days |
| **GM_A21329722** | 501667-1 | Rooted explant | Moved to fresh 150 ppm spec B5 at day 30; moved to fresh 150 ppm spec B5 at day 37; moved to 50 ppm spec B5 at day 52; shoot harvested on 150 ppm spec BRM at day 52 and explant retained; explant moved to 100 ppm spec B5 at day 83 | 99 days |

**Table 17: Summary of Plastid Transformation Metrics using pMON285270.**

| **Construct** | **Soy DEEs bombarded** | **Greening explants subcultured** | **Greening explants GFP+** | **GFP+ greening explants developing shoots with trifoliates (putative events)** | **GFP+ R0 plants to GH (actual events)** | **GFP negative R0 plants sent to GH (nuclear escapes or other)** | **pTF** |
|---|---|---|---|---|---|---|---|
| **pMON285270** | 20896 | 129 | 66 | 15 | 11 | 1 | 0.05% |

### Example 13: Comparison of Kinetin vs. TDZ, or no PGRs

An experiment was performed with pMON304331 (a construct including AFP1 as the gene of interest; and *aadA* for selection), comparing kinetin or TDZ in the preculture medium, along with a non-PGR treatment control. Greening spectinomycin resistant explants were obtained with both TDZ and kinetin preculture, but more were observed with TDZ (Table 18). No spectinomycin resistant explants were observed in this experiment without either of the PGRs in the preculture medium. In separate experiments, GFP positive explants and plants were obtained with TDZ in the preculture medium (data not shown).

**Table 18: Comparisons of Kinetin vs. TDZ. vs. no PGRs in Preculture.**

| **Preculture Media** | **# Soy DEEs** | **# Spec Resistant Phenotypes** |
|---|---|---|
| 1 day LIMS 4859 without PGRs 28°C 16/8 photoperiod | 543 | 0 |
| 1 day LIMS 4859 (MS + 1 ppm TDZ + 0.2 ppm 2,4-D) 28°C 16/8 photoperiod | 3849 | 5 |
| 1 day LIMS 4859 (replacing TDZ with 5 ppm kinetin) 28°C 16/8 photoperiod | 3338 | 1 |

### Example 14: Immature Soy Meristem Transformation and Short Selection

In a single experiment, immature meristems were obtained from manually excised green immature greenhouse-grown seed, precultured on LIMS 4859 media and bombarded with pMON285270. A putative GFP expressing event was produced by this method. Although GFP positive tissue from this event did not regenerate into a shoot, tissue samples taken from this event were used to demonstrate GFP expression by confocal microscopy, and PCR analysis was performed to show presence of the GFP expressing construct (data not shown). These data confirm the transplastomic character of this event and further demonstrate that immature embryos can be used as targets for plastid transformation according to present methods.

In separate experiments, transplastomic events were also generated using very short durations of selection (1 to 2 weeks) at 150 ppm spectinomycin following bombardment of mature soy embryo explants with pMON285270. However, removal of explants from selection after only 1 or 2 weeks resulted in a large decrease in the percentage of transformed plastids as measured by GFP expression (data not show

## Claims

1. A method of transforming a plant plastid, comprising the steps of:
(a) preparing an explant from a seed of a plant, the explant comprising meristematic tissue of a mature embryo of the seed; and
(b) transforming at least one plastid of a cell of the explant with an exogenous DNA molecule, the exogenous DNA molecule comprising:
(i) a first arm region homologous to a first plastid genome sequence;
(ii) a second arm region homologous to a second plastid genome sequence; and
(iii) an insertion sequence positioned between the first arm region and the second arm region of the exogenous DNA molecule,
wherein the cells of the explant do not form a callus tissue prior to the transforming step (b), and wherein the insertion sequence is incorporated into a plastid genome of the plant cell between the first plastid genome sequence and the second plastid genome sequence.

2. The method of claim 1, wherein the seed is dry seed comprising a mature embryo.

3. The method of claim 2, wherein the dry seed has a moisture content in a range from about 3% to about 25%.

4. The method of claim 1, further comprising:
germinating the explant after the transforming step (b).

5. The method of claim 4, further comprising:
developing a plastid transformed plant from the germinated explant.

6. The method of claim 1, wherein the insertion sequence comprises a first DNA expression cassette and a second DNA expression cassette, the first DNA expression cassette comprising a first transgene operably linked to a first plastid promoter, and the second DNA expression cassette comprising a second transgene operably linked to a second plastid promoter, wherein the first transgene confers a trait of agronomic interest when expressed in a plant, and the second transgene is a plant selectable marker gene conferring tolerance to a selection agent.

7. The method of claim 1, further comprising:
storing the explant under dry conditions for about 1 hour to about 2 years prior to the transforming step (b), wherein the explant remains viable and competent for transformation during storage.

8. The method of claim 1, further comprising:
drying the explant prior to the transforming step (b).

9. The method of claim 1, further comprising:
excising the explant from the seed of the plant.

10. A method of transforming a plant plastid, comprising the steps of:
transforming at least one plastid of a meristematic cell of a mature embryo explant of a dry seed with an exogenous DNA molecule, the exogenous DNA molecule comprising:
(i) a first arm region homologous to a first plastid genome sequence;
(ii) a second arm region homologous to a second plastid genome sequence; and
(iii) an insertion sequence positioned between the first arm region and the second arm region of the exogenous DNA molecule,
wherein the cells of the explant do not form a callus tissue prior to the transforming step, and wherein the insertion sequence is incorporated into the plastid genome of the plant cell between the first plastid genome sequence and the second plastid genome sequence.

11. The method of claim 10, further comprising:
excising the explant from a seed of a plant.

## Patentansprüche

1. Verfahren zum Transformieren eines Pflanzenplastids, umfassend die Schritte:
(a) Herstellen eines Explantats aus einem Samen einer Pflanze, wobei das Explantat meristematisches Gewebe eines reifen Embryos des Samens umfasst; und
(b) Transformieren wenigstens eines Plastids einer Zelle des Explantats mit einem exogenen DNA-Molekül, wobei das exogene DNA-Molekül Folgendes umfasst:
(i) eine erste Armregion, die zu einer ersten Plastidgenomsequenz homolog ist;
(ii) eine zweite Armregion, die zu einer zweiten Plastidgenomsequenz homolog ist; und
(iii) eine Insertionssequenz, die zwischen der ersten Armregion und der zweiten Armregion des exogenen DNA-Moleküls positioniert ist,
wobei die Zellen des Explantats vor dem Transformierschritt (b) kein Kallusgewebe bilden und wobei die Insertionssequenz in ein Plastidgenom der Pflanzenzelle zwischen der ersten Plastidgenomsequenz und der zweiten Plastidgenomsequenz eingebaut wird.

2. Verfahren nach Anspruch 1, wobei es sich bei dem Samen um einen reifen Embryo umfassenden Trockensamen handelt.

3. Verfahren nach Anspruch 2, wobei der Trockensamen einen Feuchtigkeitsgehalt in einem Bereich von etwa 3% bis etwa 25% aufweist.

4. Verfahren nach Anspruch 1, ferner umfassend:
Keimenlassen des Explantats nach dem Transformierschritt (b).

5. Verfahren nach Anspruch 4, ferner umfassend:
Entwickeln einer plastidtransformierten Pflanze aus dem gekeimten Explantat.

6. Verfahren nach Anspruch 1, wobei die Insertionssequenz eine erste DNA-Expressionskassette und eine zweite DNA-Expressionscassette umfasst, wobei die erste DNA-Expressionskassette ein erstes Transgen in operativer Verknüpfung mit einem ersten Plastidpromotor und die zweite DNA-Expressionskassette ein zweites Transgen in operativer Verknüpfung mit einem zweiten Plastidpromotor umfasst, wobei das erste Transgen bei seiner Expression in einer Pflanze ein Merkmal von agronomischem Interesse verleiht und es sich bei dem zweiten Transgen um ein selektierbares Pflanzenmarkergen handelt, das Toleranz gegenüber einem Selektionsmittel verleiht.

7. Verfahren nach Anspruch 1, ferner umfassend:
Lagern des Explantats unter trockenen Bedingungen über einen Zeitraum von etwa 1 Stunde bis etwa 2 Jahren vor dem Transformierschritt (b), wobei das Explantat während der Lagerung lebensfähig und transformationskompetent bleibt.

8. Verfahren nach Anspruch 1, ferner umfassend:
Trocknen des Explantats vor dem Transformierschritt (b).

9. Verfahren nach Anspruch 1, ferner umfassend:
Exzisieren des Explantats aus dem Samen der Pflanze.

10. Verfahren zum Transformieren eines Pflanzenplastids, umfassend die Schritte:
Transformieren wenigstens eines Plastids einer meristematischen Zelle eines reifen Embryoexplantats eines Trockensamens mit einem exogenen DNA-Molekül, wobei das exogene DNA-Molekül Folgendes umfasst:
(i) eine erste Armregion, die zu einer ersten Plastidgenomsequenz homolog ist;
(ii) eine zweite Armregion, die zu einer zweiten Plastidgenomsequenz homolog ist; und
(iii) eine Insertionssequenz, die zwischen der ersten Armregion und der zweiten Armregion des exogenen DNA-Moleküls positioniert ist,
wobei die Zellen des Explantats vor dem Transformierschritt kein Kallusgewebe bilden und wobei die Insertionssequenz in das Plastidgenom der Pflanzenzelle zwischen der ersten Plastidgenomsequenz und der zweiten Plastidgenomsequenz eingebaut wird.

11. Verfahren nach Anspruch 10, ferner umfassend:
Exzisieren des Explantats aus einem Samen einer Pflanze.

## Revendications

1. Procédé de transformation d'un plaste de plante, comprenant les étapes de :
(a) préparation d'un explant à partir d'une graine d'une plante, l'explant comprenant un tissu méristématique d'un embryon mature de la graine ; et
(b) transformation d'au moins un plaste d'une cellule de l'explant avec une molécule d'ADN exogène, la molécule d'ADN exogène comprenant :
(i) une première région-bras homologue à une première séquence du génome de plaste ;
(ii) une deuxième région-bras homologue à une deuxième séquence du génome de plaste ; et
(iii) une séquence d'insertion positionnée entre la première région-bras et la deuxième région-bras de la molécule d'ADN exogène,
dans lequel les cellules de l'explant ne forment pas un tissu de cal avant l'étape de transformation (b), et dans lequel la séquence d'insertion est incorporée dans un génome de plaste de la cellule de plante entre la première séquence du génome de plaste et la deuxième séquence du génome de plaste.

2. Procédé de la revendication 1, dans lequel la graine est une graine sèche comprenant un embryon mature.

3. Procédé de la revendication 2, dans lequel la graine sèche a une teneur d'humidité comprise dans une plage de 3 % environ jusqu'à 25 % environ.

4. Procédé de la revendication 1, comprenant en outre :
une germination de l'explant après l'étape de transformation (b).

5. Procédé de la revendication 4, comprenant en outre :
un développement d'une plante aux plastes transformés à partir de l'explant germé.

6. Procédé de la revendication 1, dans lequel la séquence d'insertion comprend une première cassette d'expression d'ADN et une deuxième cassette d'expression d'ADN, la première cassette d'expression d'ADN comprenant un premier transgène lié, de manière opérationnelle, à un premier promoteur de plaste, et la deuxième cassette d'expression d'ADN comprenant un deuxième transgène lié, de manière opérationnelle, à un deuxième promoteur de plaste, dans lequel le premier transgène confère un trait d'intérêt agronomique quand il est exprimé dans une plante et le deuxième transgène est un gène marqueur sélectionnable de plante conférant une tolérance à un agent de sélection.

7. Procédé de la revendication 1, comprenant en outre : un stockage de l'explant dans des conditions sèches pendant 1 heure environ jusqu'à 2 ans environ avant l'étape de transformation (b), dans lequel l'explant reste viable et compétent pour une transformation durant le stockage.

8. Procédé de la revendication 1, comprenant en outre :
un séchage de l'explant avant l'étape de transformation (b).

9. Procédé de la revendication 1, comprenant en outre :
une excision de l'explant à partir de la graine de la plante.

10. Procédé de transformation d'un plaste de plante, comprenant les étapes de :
transformation d'au moins un plaste d'une cellule méristématique d'un explant de type embryon mature issu d'une graine sèche avec une molécule d'ADN exogène, la molécule d'ADN exogène comprenant :
(i) une première région-bras homologue à une première séquence du génome de plaste ;
(ii) une deuxième région-bras homologue à une deuxième séquence du génome de plaste ; et
(iii) une séquence d'insertion positionnée entre la première région-bras et la deuxième région-bras de la molécule d'ADN exogène,
dans lequel les cellules de l'explant ne forment pas un tissu de cal avant l'étape de transformation, et dans lequel la séquence d'insertion est incorporée dans le génome de plaste de la cellule de plante entre la première séquence du génome de plaste et la deuxième séquence du génome de plaste.

11. Procédé de la revendication 10, comprenant en outre :
une excision de l'explant à partir d'une graine d'une plante.
